# EUROPEAN PATENT SPECIFICATION

(11) **EP 3 668 319 B1**
(45) Date of publication and mention of the grant of the patent: **25.03.2026**
(21) Application number: 18846662.7
(22) Date of filing: 15.08.2018
(51) Int. Cl.: A01N 63/00, A61K 35/12, C12N 1/00, C12N 5/02, A61K 35/28

(54) **PURIFIED MESENCHYMAL STEM CELL EXOSOMES AND USES THEREOF**
GEREINIGTE EXOSOME AUS MESENCHYMALEN STAMMZELLEN UND VERWENDUNGEN DAVON
EXOSOMES DE CELLULES SOUCHES MÉSENCHYMATEUSES PURIFIÉES ET LEURS UTILISATIONS

(30) Priority: 15.08.2017 US 201762545610 P
(43) Date of publication of application: 24.06.2020
(73) Proprietor: Children's Medical Center Corporation, Boston, MA 02115 (US)
(72) Inventor: MITSIALIS, S., Alexander, Newton, MA 02459 (US); KOUREMBANAS, Stella, Newton, MA 02459 (US)
(74) Representative: HGF
(86) International application number: PCT/US2018/000144
(87) International publication number: WO 2019/035880

(56) References cited:
- WO-A1-2012/125471
- WO-A1-2015/179227
- WO-A1-2016/043654
- US-A1- 2015 190 430
- US-A1- 2016 220 613
- TH�BAUD BERNARD ET AL: "Bronchopulmonary dysplasia", NATURE REVIEWS DISEASE PRIMERS, NATURE PUBLISHING GROUP UK, LONDON, vol. 5, no. 1, 14 November 2019 (2019-11-14), XP037114995, [retrieved on 20191114], DOI: 10.1038/S41572-019-0127-7
- DATABASE BIOSIS [online] BIOSCIENCES INFORMATION SERVICE, PHILADELPHIA, PA, US; 1 April 2017 (2017-04-01), WILLIS GARETH R ET AL: "Mesenchymal Stem Cell Exosome Treatment Restores Lung Architecture and Ameliorates Pulmonary Hypertension Associated with Bronchopulmonary Dysplasia", XP002802523, Database accession no. PREV201700750539
- FASEB JOURNAL, vol. 31, no. Suppl. 1, April 2017 (2017-04-01), EXPERIMENTAL BIOLOGY MEETING; CHICAGO, IL, USA; APRIL 22 -26, 2017, pages 327.5, ISSN: 0892-6638(print)
- GARETH R. WILLIS ET AL: "Mesenchymal Stromal Cell Exosomes Ameliorate Experimental Bronchopulmonary Dysplasia and Restore Lung Function through Macrophage Immunomodulation", vol. 197, no. 1, 30 August 2017 (2017-08-30), US, pages 104 - 116, XP055692139, ISSN: 1073-449X, Retrieved from the Internet <URL:https://www.atsjournals.org/doi/full/10.1164/rccm.201705-0925OC> DOI: 10.1164/rccm.201705-0925OC
- LOU ET AL.: "Mesenchymal stem cell -derived exosomes as a new therapeutic strategy for liver diseases", EXP MOL MED., vol. 49, no. 6, 16 June 2017 (2017-06-16), pages e346, XP055577647
- LONG ET AL.: "Intranasal MSC-derived A1-exosomes ease inflammation, and prevent abnormal neurogenesis and memory dysfunction after status epilepticus", PROC NATL ACAD SCI USA., vol. 114, no. 17, April 2017 (2017-04-01), pages E3536 - E3545, XP055481659, DOI: doi:10.1073/pnas.1703920114
- COLLINO ET AL.: "Exosome and Microvesicle-Enriched Fractions Isolated from Mesenchymal Stem Cells by Gradient Separation Showed Different Molecular Signatures and Functions on Renal Tubular Epithelial Cells", STEM CELL REV., vol. 13, no. 2, April 2017 (2017-04-01), pages 226 - 243, XP055544071, DOI: doi:10.1007/s12015-016-9713-1
- TANNETTA ET AL.: "Characterisation of Syncytiotrophoblast Vesicles in Normal Pregnancy and Pre-Eclampsia: Expression of Fit-1 and Endoglin", PLOS ONE, vol. 8, no. 2, 20 February 2013 (2013-02-20), pages e56754, XP055577658
- DONNARUMMA ET AL.: "Cancer-associated fibroblasts release exosomal microRNAs that dictate an aggressive phenotype in breast cancer", ONCOTARGET, vol. 8, no. 12, 21 March 2017 (2017-03-21), pages 19592 - 19608, XP055577657

## Description

### BACKGROUND

The therapeutic capacity of mesenchymal stem/stromal cells (MSCs) in different organs (e.g., the lung) has been shown to be comprised in their secretome (e.g., in the exosomes they release). Beneficial properties of MSC exosome treatment have also been reported in a number of disease models, including myocardial infarction, kidney injury, allergy & similar immune-modulating conditions, and neurological protection. However, frequently, crude exosome isolation techniques coupled with poor analytical characterization diminishes the therapeutic impact of MSC exosomes, impairing bioavailability and co-contaminating preparations with non-exosome material.

WILLIS GARETH R ET AL: "Mesenchymal Stem Cell Exosome Treatment Restores Lung Architecture and Ameliorates Pulmonary Hypertension Associated with Bronchopulmonary Dysplasia", Database accession no. PREV201700750539 ; & FASEB JOURNAL, vol. 31, no. Suppl. 1, April 2017 (2017-04), page 327.5, EXPERIMENTAL BIOLOGY MEETING; CHICAGO, IL, USA; APRIL 22-26, 2017 discloses the purification and characterization of MSC-exosomes/extracellular vesicles (MSC-EV) from both human bone marrow (BMSC) and umbilical cord Wharton's jelly (WJ) and investigates the efficacy of MSC-EV treatments in BPD.

WO 2015/179227 A1 discloses compositions comprising exosome subpopulations, and methods of their use in subjects having certain disorders including lung disorders, cardiovascular disorders, renal disorders and ischemic neural disorders.

WO 2012/125471 A1 describes compositions comprising mesenchymal stem cell (MSC) derived exosomes and methods of their use in subjects having certain lung diseases including inflammatory lung disease.

WO 2016/043654 A1 describes the use of exosome such as mesenchymal stem cell exosomes in a method of promoting, restoring or enhancing homeostasis in an individual suffering from graft versus host disease (GVHD) or epidermolysis bullosa (EB).

### SUMMARY

The presently claimed invention is as defined in the claims.

Described herein are the characterization of purified MSC exosomes (e.g., from human umbilical cord Wharton's Jelly or bone marrow). Using the purification methods described herein, MSC exosomes that afford therapeutic efficacy to lung diseases (e.g., Bronchopulmonary dysplasia (BPD)) are isolated and markers associated with such purified MCS exosomes are identified. Further described herein are methods of treating lung diseases (e.g., BPD) using the purified MSC exosomes. In some embodiments, a bolus dose of MSC exosome is effective in reducing the severity of a lung disease (e.g., BPD).

Accordingly, some aspects of the present invention provide a pharmaceutical composition for treating hyperoxia-induced lung injury, hyperoxia-induced pulmonary hypertension, bronchopulmonary dysplasia, and reducing pulmonary fibrosis, comprising an isolated mesenchymal stem cell (MCS) exosome, wherein: (i) the isolated MSC exosome comprises four or more markers selected from the group consisting of FLT1, PLAUR, MME, CDH2, SLC16A3, CDH13, ITGB5, ITGA11, APP, GPC6, IGSF8, IRP2, TSPAN9, DAG1, SLC38A2, SLC12A8, GJA1, ITGA1, PLXNB2, SLC16A1, and PROCR; (ii) the isolated MSC exosome comprises one or more markers that is enriched compared to a fibroblast exosome, the one or more markers selected from the group consisting of: PTk7, CD109, SLC1A5, ITGA3, ITGA2, BSG, DPP4, ATP1A1, FAP, VASN, IGF2R, and CD82; and/or (iii) the isolated MSC exosome comprises one or more markers that is enriched compared to a fibroblast exosome, the one or more markers selected from the group consisting of RCN1, RAP1B, GDF15, FLT1, OLFML3, PLOD2, PSMA4, PAPPA, INHBA, SPOCK1, HSPB1, LOXL4, POLD3, CALU, IGFBP4, C4B, TINAGL1, SULF1, TPM3, AHNAK, CALR, RRAS2, PFKP, and COL16A1, wherein the isolated mesenchymal stem cell (MCS) exosome is obtained by a method comprising: a) obtaining MSC-CM from WJMSCs, BMSCs or human dermal fibroblasts (HDFs) cultures; b) concentrating the MSC-CM; c) fractionating the concentrated MSC-CM using an iodixanol (IDX) cushion gradient; wherein the fractionating comprises floating the MSC-CM on the IDX cushion gradient and ultracentrifugation; d) collecting a fraction that floats in the gradient at a density of -1.18g/ml; and e) extracting exosomes from the collected fraction; thereby isolating the mesenchymal stem cell (MCS) exosome.

In some embodiments, the isolated MSC exosome comprises four or more markers selected from the group consisting of FLT1, PLAUR, MME, CDH2, SLC16A3, CDH13, ITGB5, ITGA11, APP, GPC6, IGSF8, IRP2, TSPAN9, DAG1, SLC38A2, SLC12A8, GJA1, ITGA1, PLXNB2, SLC16A1, and PROCR.

In some embodiments, the isolated MSC exosome comprises one or more markers that is enriched compared to a fibroblast exosome, the one or more markers selected from the group consisting of: PTk7, CD109, SLC1A5, IGTA3, ITGA2, BSG, DPP4, ATP1A1, FAP, VASN, IGF2R, and CD82.

In some embodiments, the isolated MSC exosome comprises one or more markers that is enriched compared to a fibroblast exosome, the one or more markers selected from the group consisting of RCN1, RAP1B, GDF15, FLT1, OLFML3, PLOD2, PSMA4, PAPPA, INHBA, SPOCK1, HSPB1, LOXL4, POLD3, CALU, IGFBP4, C4B, TINAGL1, SULF1, TPM3, AHNAK, CALR, RRAS2, PFKP and COL16A1.

In some embodiments, the isolated MSC exosome further comprises one or more markers selected from the group consisting of: FLOT1, CD9, and CD63.

In some embodiments, the isolated MSC exosome is isolated from MSC-conditioned media. In some embodiments, the MSC is from Warton's Jelly or bone marrow. In some embodiments, the isolated MSC exosome is substantially free of non-exosomal protein contaminants. In some embodiments, the isolated MSC exosome has a diameter of about 30-150 nm. In some embodiments, the isolated MSC exosome has immunomodulatory activity.

Further provided herein are pharmaceutical compositions comprising the isolated MSC exosomes described herein. In some embodiments, the pharmaceutical composition further comprises a secondary agent. In some embodiments, the secondary agent is a pulmonary surfactant, a steroid, an antioxidant, or inhaled nitric oxide. In some embodiments, the pharmaceutical composition further comprises a pharmaceutically acceptable carrier.

Other aspects of the present disclosure provide
a pharmaceutical composition according to the present invention, for use in treating hyperoxia-induced lung injury, hyperoxia-induced pulmonary hypertension, bronchopulmonary dysplasia, and reducing pulmonary fibrosis in a subject.

In some embodiments, the subject is a human subject. In some embodiments, the human subject was born before 37 weeks of gestation. In some embodiments, the human subject was born before 26 weeks of gestation. In some embodiments, the subject has hyperoxia-induced lung injury. In some embodiments, the subject has been administered oxygen or has been on a ventilator. In some embodiments, the subject has or is at risk of developing bronchopulmonary dysplasia (BPD). In some embodiments, the subject has hyperoxia-induced pulmonary hypertension. In some embodiments, the subject exhibits peripheral pulmonary arterial remodeling. In some embodiments, the subject has inflammation in the lung.

In some embodiments, the subject is less than four weeks of age. In some embodiments, the subject is 3-18 years of age. In some embodiments, the subject is an adult.

In some embodiments, the isolated MSC exosome is administered in a bolus dose to the subject. In some embodiments, the isolated MSC exosome is administered repeatedly to the subject.

In some embodiments, the isolated MSC exosome improves lung function. In some embodiments, the isolated MSC exosome restores lung architecture. In some embodiments, the isolated MSC exosome reduces pulmonary fibrosis. In some embodiments, the isolated MSC exosome reduces inflammation in the lung. In some embodiments, the isolated MSC exosome reduces peripheral pulmonary arterial remodeling.

In some embodiments, the isolated MSC exosome is administered within an hour of birth. In some embodiments, the isolated MSC exosome is administered within one month of birth.

In some embodiments, the isolated MSC exosome is administered intravenously or intranasally. In some embodiments, the isolated MSC exosome is administered to the lung or trachea of the subject. In some embodiments, the isolated MSC exosome is administered by inhalation. In some embodiments, the isolated MSC exosome is administered in an aerosol. In some embodiments, the isolated MSC exosome is administered using a nebulizer. In some embodiments, the isolated MSC exosome is administered using an intratracheal tube.

In some embodiments, the subject is a mammal. In some embodiments, the mammal is a human. In some embodiments, the mammal is a rodent. In some embodiments, the rodent is a mouse or a rat.

The isolated mesenchymal stem cell (MSC) exosome the pharmaceutical composition described herein may also be used in the manufacturing of a medicament for treating a lung disease.

Other aspects of the present disclosure provide methods of isolating a mesenchymal stem cell (MSC) exosome, the method comprising fractionating MSC-conditioned media using an iodixanol (IDX) cushion gradient and collecting a fraction that is substantially free of non-exosomal protein contaminants. In some embodiments, the MSC-conditioned media is concentrated prior to fractionation. In some embodiments, the MSC-conditioned media is concentrated via tangential flow filtration. In some embodiments, the fractionating comprises floating the MSC-conditioned media on the IDX cushion gradient and ultracentrifugation.

MSC exosomes isolated using the methods of isolation described herein are also described.

The summary above is meant to illustrate, in a non-limiting manner, some of the embodiments, advantages, features, and uses of the technology disclosed herein. Other embodiments, advantages, features, and uses of the technology disclosed herein will be apparent from the Detailed Description, the Drawings, the Examples, and the Claims.

### BRIEF DESCRIPTION OF THE DRAWINGS

The invention is defined in the claims. Any of the following Figures that do not fall within the scope of the claims do not form part of the claimed invention and are provided for comparative purposes only.

The accompanying drawings are not intended to be drawn to scale. In the drawings, each identical or nearly identical component that is illustrated in various figures is represented by a like numeral. For purposes of clarity, not every component may be labeled in every drawing. The patent or application file contains at least one drawing executed in color. Copies of this patent or patent application publication with color drawing(s) will be provided by the Office upon request and payment of the necessary fee. In the drawings:

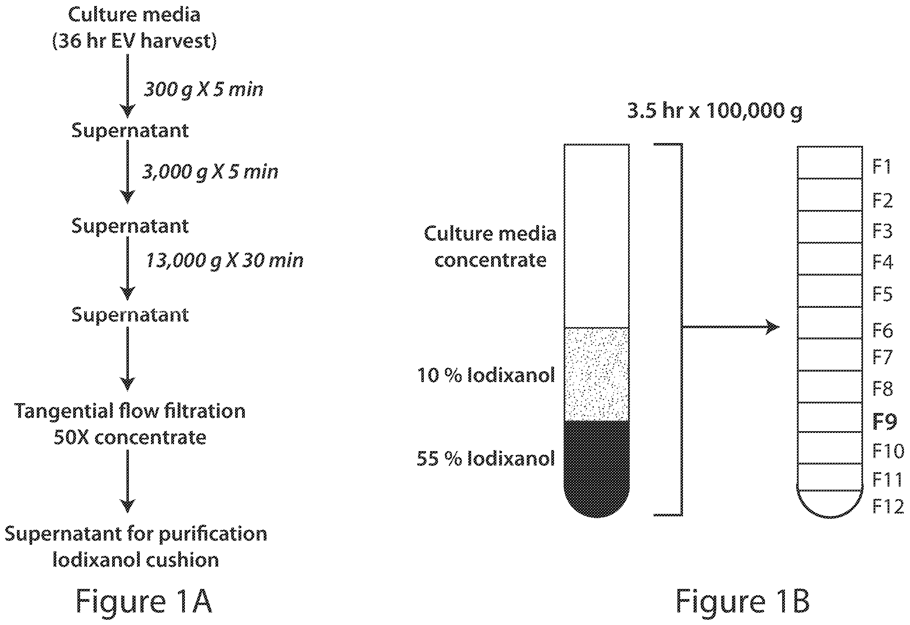

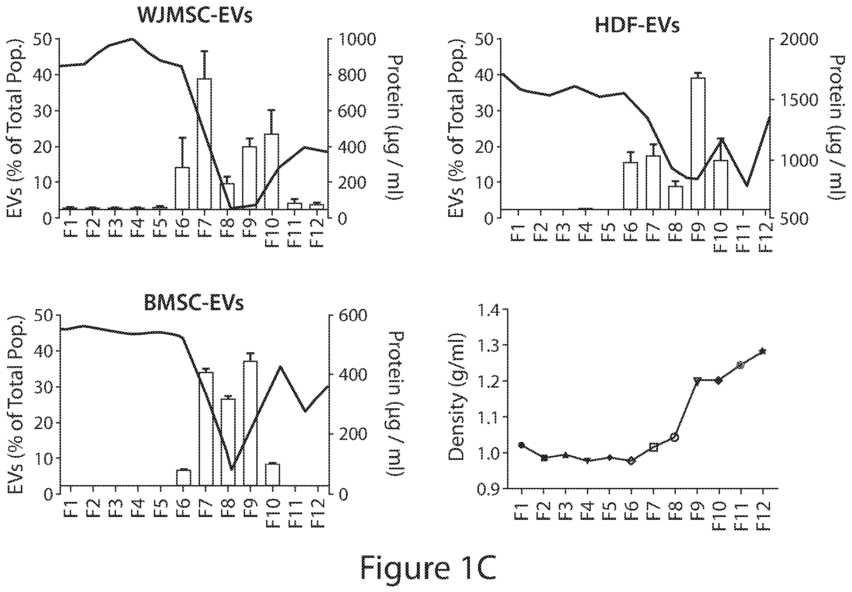

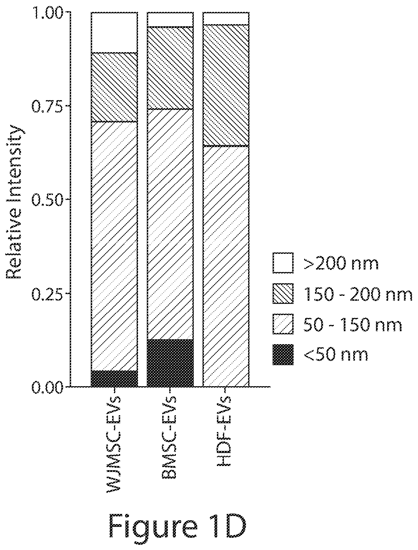

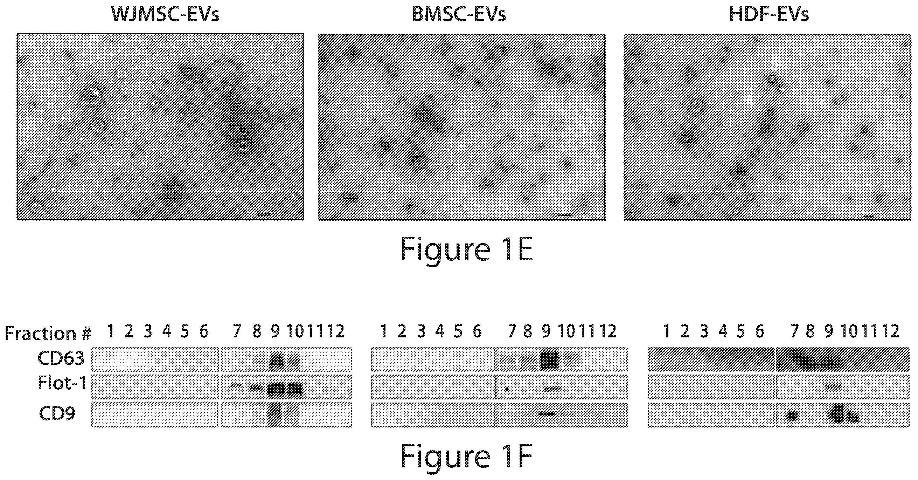

**Figures 1A to 1F****. Purification, isolation, and characterization of exosomes (EVs). (****Figure 1A****)** WJMSCs, BMSCs, and HDFs secrete heterogeneous EVs that can be isolated by successive differential centrifugation coupled and concentrated through tangential flow filtration. **(****Figure 1B****)** Concentrated (50×) conditioned media (CM) was floated on an iodixanol (IDX) cushion gradient to purify and isolate the EV population (fraction 9, density -1.18 g/ml). **(Figure 1C)** Nanoparticle tracking analysis (NTA) and protein concentration was used to assess EV concentration and particle: protein ratio in the IDX cushion (12 × 1 ml fractions), respectively. The line denotes protein concentration and the bar charts reflect nanoparticle concentration, as per NTA. Fraction density is expressed as g/ml. **(****Figure 1D****)** Representative size distribution of WJMSC-EVs, BMSC-EVs, and HDF-EVs isolated from their corresponding fraction 9 gradients. (**Figure 1E**) Transmission electron microscopy (TEM) images demonstrating heterogeneous EV morphology (high magnification, ×30,000, scale bar = 100 nm). **(****Figure 1F****)** The IDX cushion gradient fractions were analyzed by Western blot (fraction 1-6 and 7-12, side-by-side), using antibodies to proteins representing the exosomal markers flotillin (FLOT-1) and tetraspanins CD63 and CD9. An equivalent volume of each fraction was loaded per lane and representative images are shown.

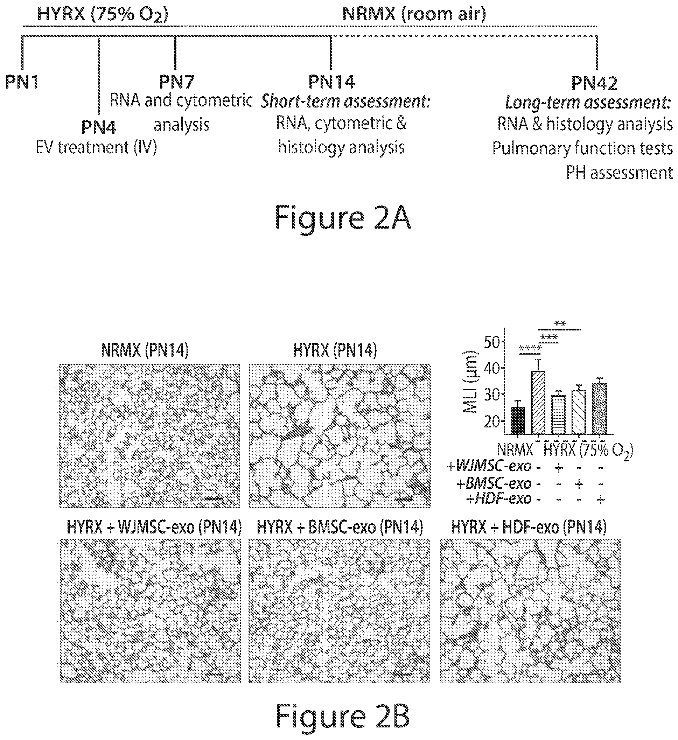

**Figures 2A to 2B****. MSC-EV treatment improves short-term lung architecture outcome.** Newborn FVB mice were exposed to HYRX (75% O₂) for 7 days. HYRX-exposed mice were compared to mice that remained at NRMX (room air). EV-treatments were delivered intravenously (IV) at PN4. Short-term outcomes were assessed at PN14 (schematic shown in **Figure 2A). (Figure 2B****)** Harvested lung sections were stained for hematoxylin and eosin (H&E). Inserts were taken at 200× magnification. HYRX-control and HDF-EV treated mice presented with significant alveolar simplification, characterized by large airspaces and incomplete alveolarization. HYRX exposed animals that received a bolus dose of either WJMSC-EVs or BMSC-EVs had a markedly improved lung alveolarization when compared to the emphysema-like appearance of the HYRX-control mice. Quantification of mean liner intercept (MLI, µm) represents a surrogate of average air space diameter. Data represents results from 3 individual studies. Mean ± SEM, n = 5 - 15 per group, ** p < 0.01, *** p < 0.001. Scale bar = 100 µm.

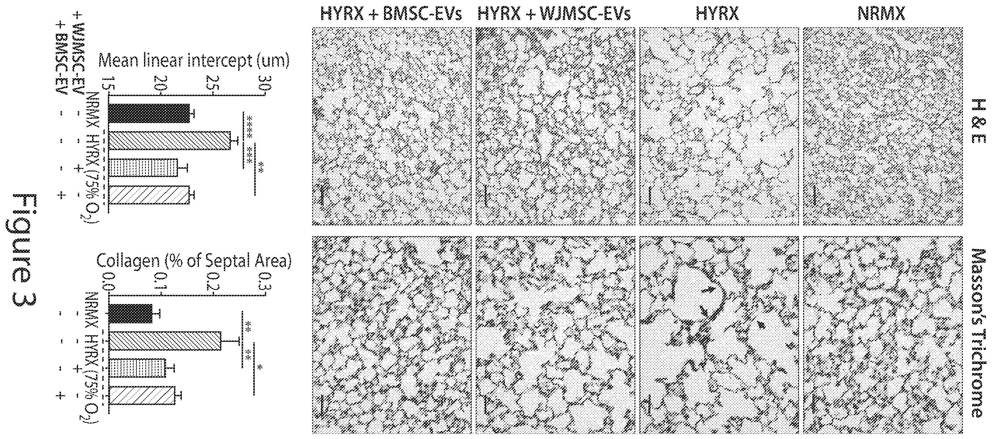

**Figure 3****. MSC-EV treatment improves lung architecture and reduces HYRX-induced fibrosis.** For long-term outcomes, mice were assessed at PN42 (6 weeks). Lung sections were stained for H&E and Masson's Trichrome to assess the degree of alveolar simplification and fibrosis, respectively. Sections were captured at 100× (H&E) and 200× (Masson's Trichrome) magnification using a Nikon Eclipse 80i microscope to illustrate extensive alveolar simplification as well as modest lung fibrosis, denoted by collagen deposition in the septal area. Compared to the emphysema-like appearance of HYRX-control animals, mice that received WJMSC-EVs or BMSC-EVs presented with a restored alveolar development that was akin to the NRMX-controls (H&E stain). Assessing collagen deposition, HYRX-controls had a modest but significant increase in septal collagen deposition when compared to NRMX-controls, as highlighted by arrows (Masson's trichrome stain). Levels were restored to NRMX-controls in HYRX-exposed mice that received either WJMSC-EVs or BMSC-EVs. Quantification of alveolar simplification was performed by measuring the mean liner intercept (MLI, µm). Collagen deposition was used as a surrogate of fibrosis and was reported as % of septal area. Mean ± SEM, n = 4-5 per group, * p < 0.05, ** p < 0.01, ***p < 0.001, **** p < 0.0001. H&E scale bar = 100 µm. Masson's Trichrome scale bar = 50 µm.

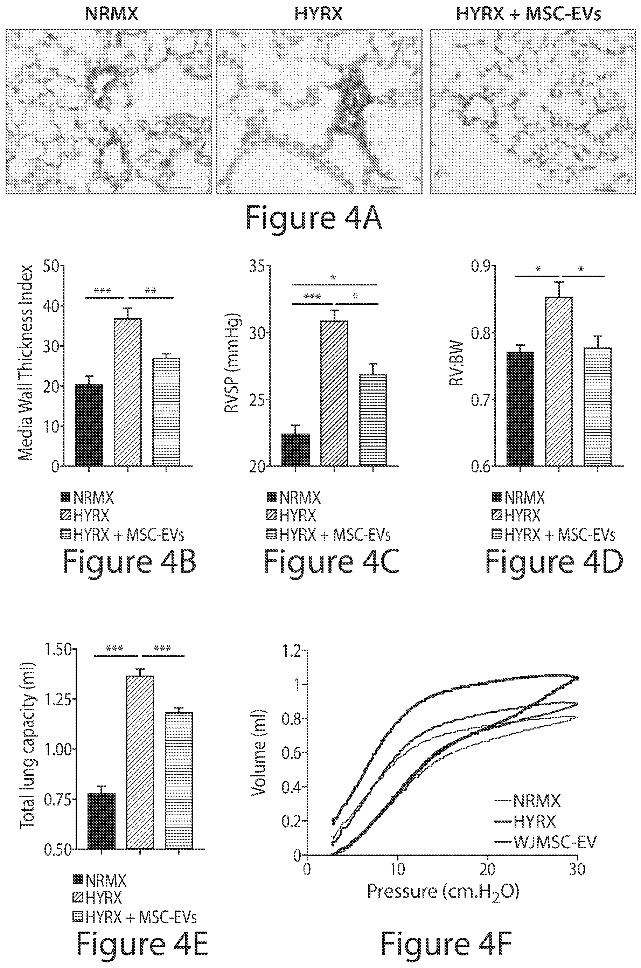

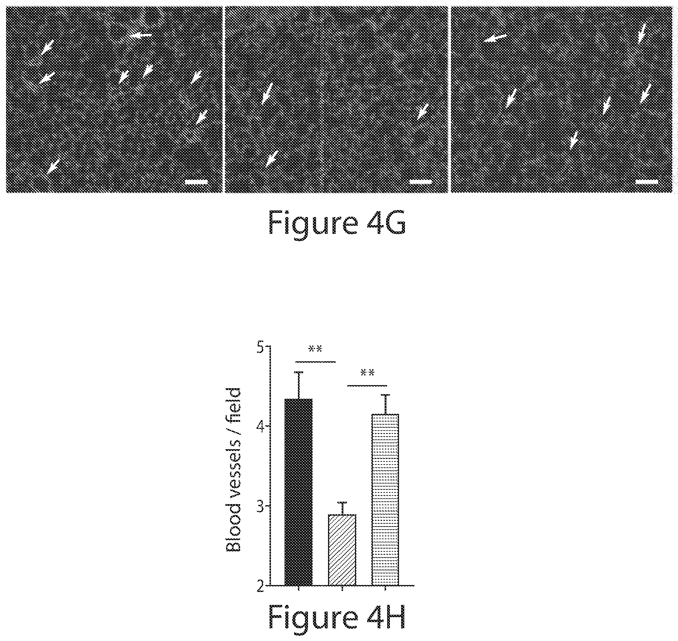

**Figures 4A to 4F****. MSC-EV administration ameliorates pulmonary hypertension, rescues peripheral pulmonary arterial remodeling, and improves lung function following hyperoxia-induced lung injury.** With minimal evidence to suggest differences between WJMSC-exo and BMSC-exo treatments, regarding their beneficial effects on long-term lung architecture, fibrosis and pulmonary vasculature, our exosome treatments (WJMSC-EV and BMSC-EV) were plotted together and referred to as MSC-EV. To assess the potential impact of MSC-EVs on HYRX-induced peripheral pulmonary vascular remodeling, lung sections of mice harvested at PN42 were stained with α-smooth muscle actin (α-SMA). **(****Figure 4A****)** Increased pulmonary vascular remodeling was evident in HYRX-controls compared to their NRMX-counterparts and MSC-EV treatment blunted the HYRX-induced increase in pulmonary vascular remodeling. Inserts were taken at 200× magnification. **(****Figure 4B****)** Media wall thickness index = 100 × (area_{[ext]} - area_{[int]}) / area_{[ext]} ). Area_{[ext]} and area_{[int]} denote the areas within the external and internal boundaries of the α-SMA layer, respectively. Mean ± SEM, *n* = 4-5 per group. Scale bar = 50 µm. **(****Figure 4C****)** To quantify the degree of pulmonary hypertension, direct RVSP measurements were taken. **(****Figure 4D****)** assessment of right ventricle to body weight (RV:BW) ratio. Mean ± SEM, *n* = 6-12 per group. **(****Figures 4E-4F****)** Pulmonary function tests were performed at postnatal day 42. **(****Figure 4E****)** Total lung capacity was significantly improved by MSC-EV treatment. **(****Figure 4F****)** Pressure-volume (PV) relationships depict representative PV-loops and demonstrate a significant shift upwards and to the left for the HYRX-control animals, indicative of emphysema-like features of lung disease and air trapping when compared to NRMX-controls. HYRX-exposed animals that received a single dose of MSC-EVs showed a significant shift in PV-loops downwards and to the right **(****Figure 4F****).** Mean ± SEM, n = 5-8 per group, *p < 0.05, **p < 0.01, ***p < 0.001. **(****Figure 4G****)** MSC-exos prevent HYRX-induced loss of blood vessels in the peripheral microvasculature. Lung sections were stained for von Willebrand factor (vWF) in mice harvested at PN42. vWF-positive vessels between 25 and 150 µm outer diameter were counted at 100× magnification in 8-12 random views. Representative immunofluorescence staining shows a significant loss of small vessels (<50 µm diameter) in HYRX-exposed compared to NRMX-control mice, and a protective effect of MSC-exos in small (< 50 µm) vessel number. Mean ± SEM, n = 5-10 per group. Scale bar = 100 µm. Arrows highlight vWF-stained pulmonary vessels. **(****Figure 4H****)** Quantification of the results obtain in Figure 4G.

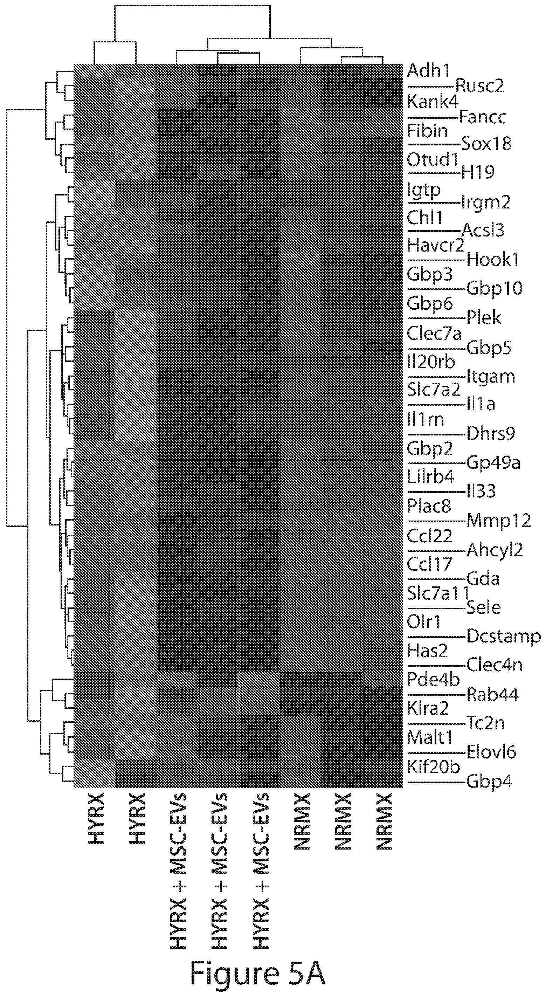

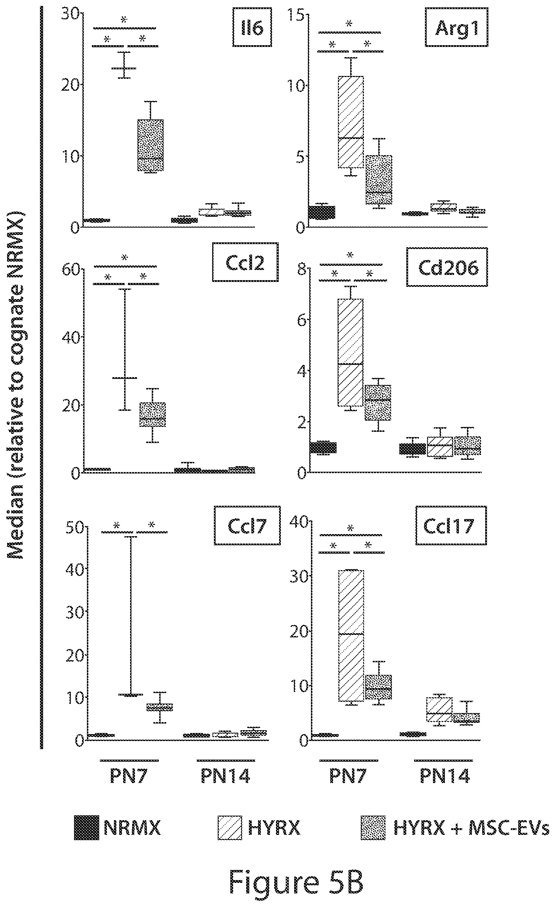

**Figures 5A to 5B****. WJMSC-EV treatment modulates hyperoxic lung transcriptome and blunts HYRX-induced inflammation.** Poly-A capture RNAseq of total mouse lung mRNA at PN7 was carried out on a subset of NRMX control (n = 3), HYRX control animals (n = 2) and WJMSC-EV treated animals (n = 3). (**Figure 5A**) Heat map cataloguing the top 50 differentially expressed genes ranked by adjusted p-value at PN7. (**Figure 5B**) Validation of select gene profiles demonstrating suppression of proinflammatory makers by MSC-EV treatment, as assessed by RT-qPCR in separate experiments. Here, results of exosome treatments (WJMSC-exosome and BMSC-exosome) were plotted together and referred to as MSC-EV. Box and whiskers plots represent median (min-max), n = 3-8 per group, * p < 0.05.

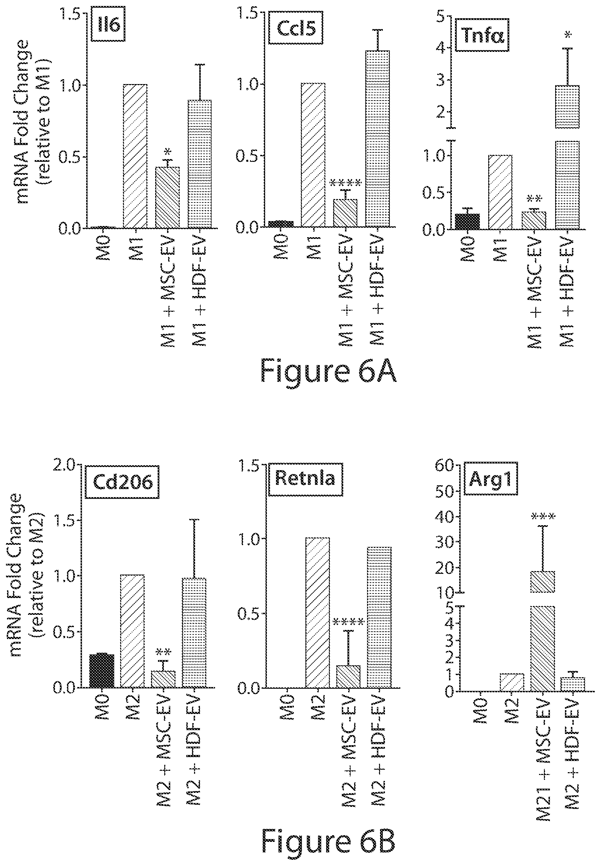

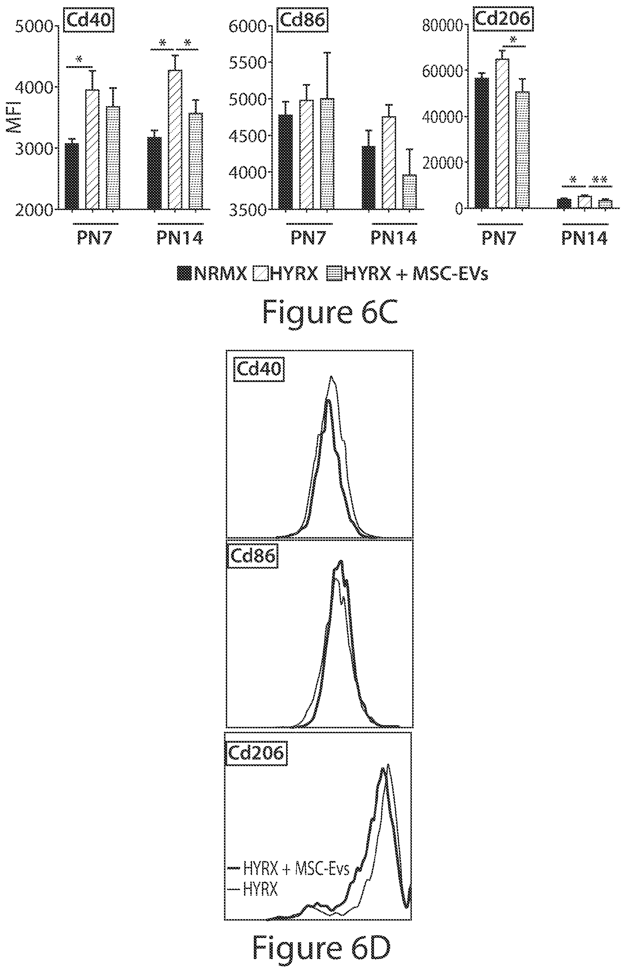

**Figures 6A to 6D****. Immunomodulatory capacity of MSC-EVs: Macrophage polarization potency assay.** Macrophage polarization plays an important role in regulating the immune response and inflammation in the developing lung. **(****Figure 6A****)** The addition of WJMSC-EVs to mouse bone marrow derived macrophages (BMDM) or alveolar macrophages (MH-S cells) polarized to the proinflammatory M1 phenotype reduced the mRNA induction of markers such as I16, Ccl5 and TNFα. **(****Figure 6B****)** The addition of WJMSC-EVs to alternatively (M2) polarized macrophages super- induced arginase-1 (Arg-1) mRNA and modulated Resistin-like alpha (Retnla) and Cd206 induction. Flow cytometry was used to assess lung macrophage(Cd45^{+ve}, Cd11b^{-ve}, Cd11c^{+ve}, Cd64^{+ve}) phenotype. **(****Figure 6C****)** M1 makers Cd40 and Cd86 as well as M2 marker Cd206 were assessed at PN7 and PN14. Median fluorescence intensity (MFI) was recorded. **(****Figure 6D****)** Representative histograms for Cd40, Cd86 and Cd206 **(****Figure 6D****).** Mean ± SEM, n = 4-8 per group, * p < 0.05, ** p <0.01, *** p < 0.001, **** p < 0.0001.

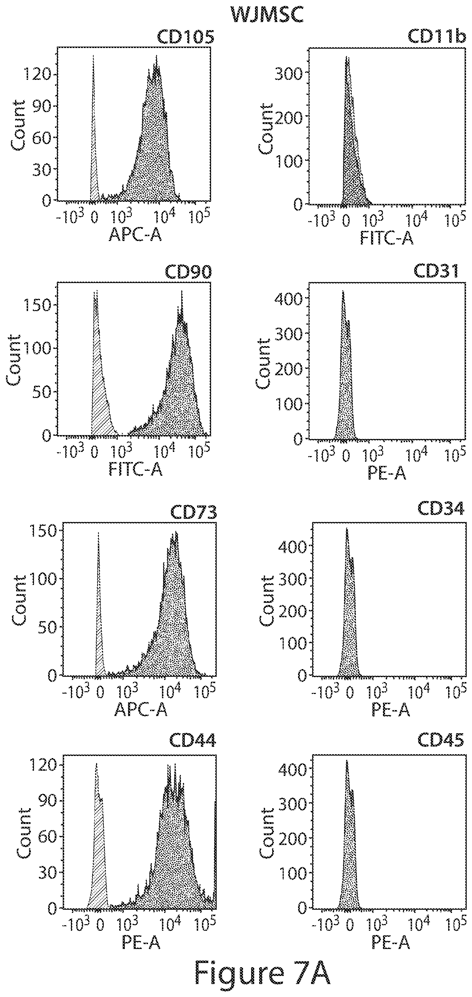

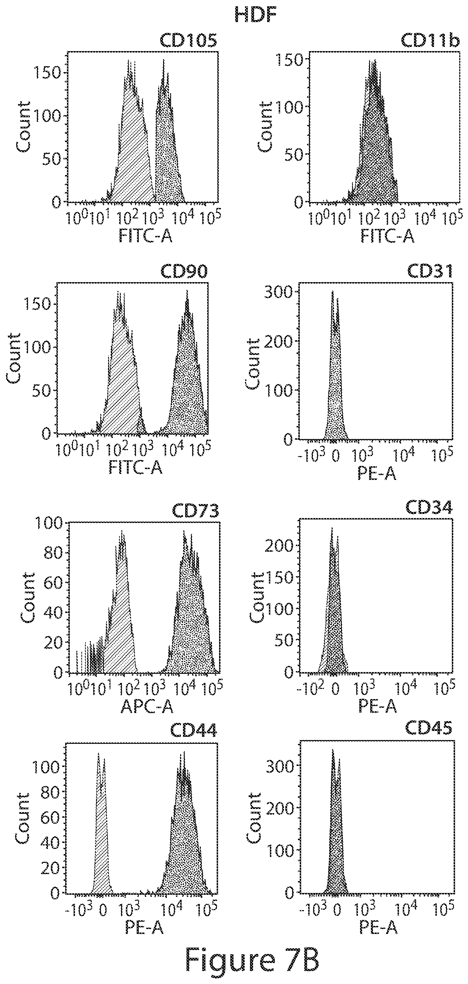

**Figures 7A to 7B****. Cytometric analysis of cell surface-markers.** Representative histograms from cytometric analysis of cells at passage 4. Both human umbilical cord WJMSCs **(****Figure 7A****)** and human dermal fibroblasts (HDFs) **(****Figure 7B****)** express CD105, CD90, CD73 and CD44, but lack expression of CD11b, CD31, CD34 and CD45. Unstained controls are shown in the lighter grey histogram, conjugated antibody-stained cells in darker grey.

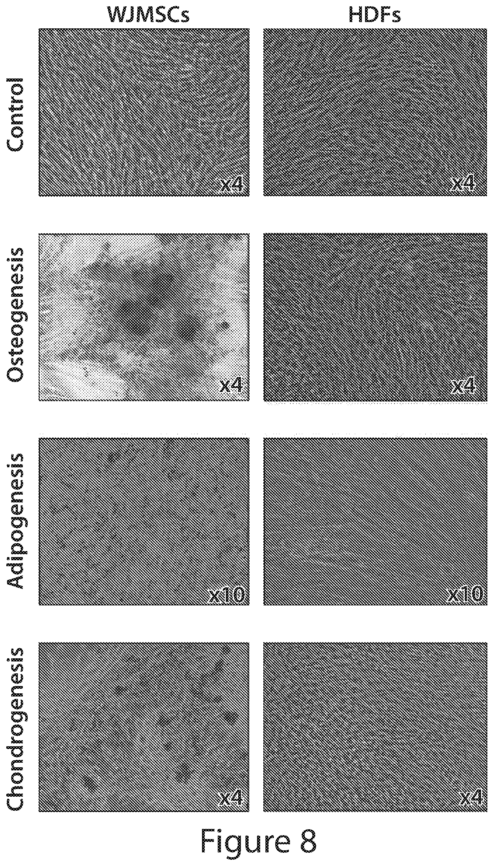

**Figure 8****. Mesenchymal stem cell differentiation capacity.** The *in vitro* differentiation potential of WJMSCs and HDF cultures to osteocyte, adipocyte and chondrocyte lineages was assessed using commercially purchased kits. In contrast to HDFs, WJMSCs displayed calcium deposition (visualized using Alizarin Red S staining), lipid droplets (*adipogenesis,* which were then stained using Oil Red) and positive chondrocyte formation (alcian blue staining) when subjected to the respective differentiation media.

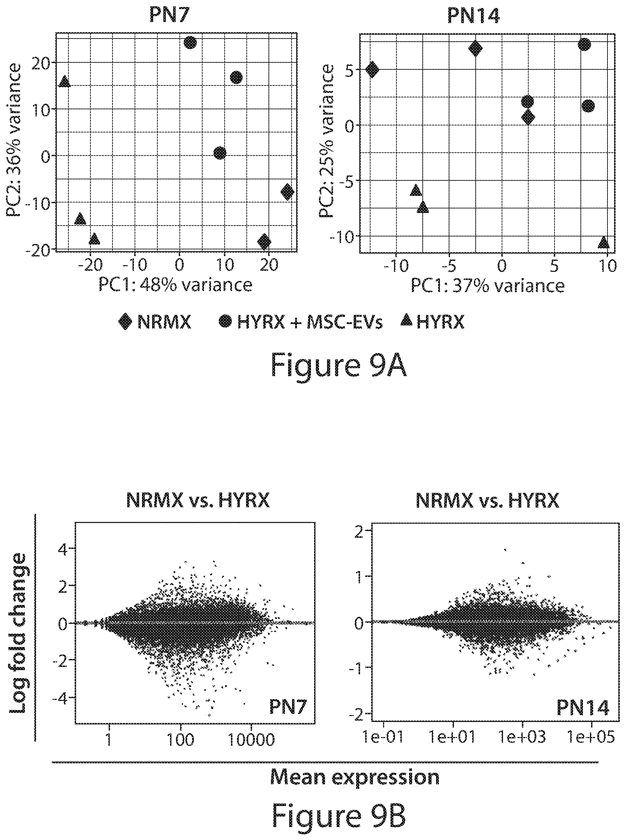

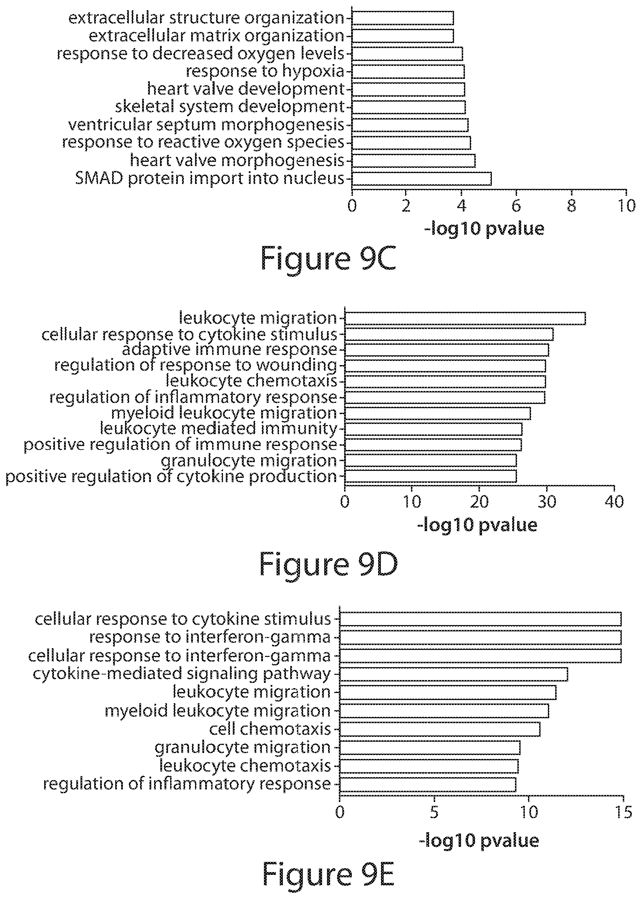

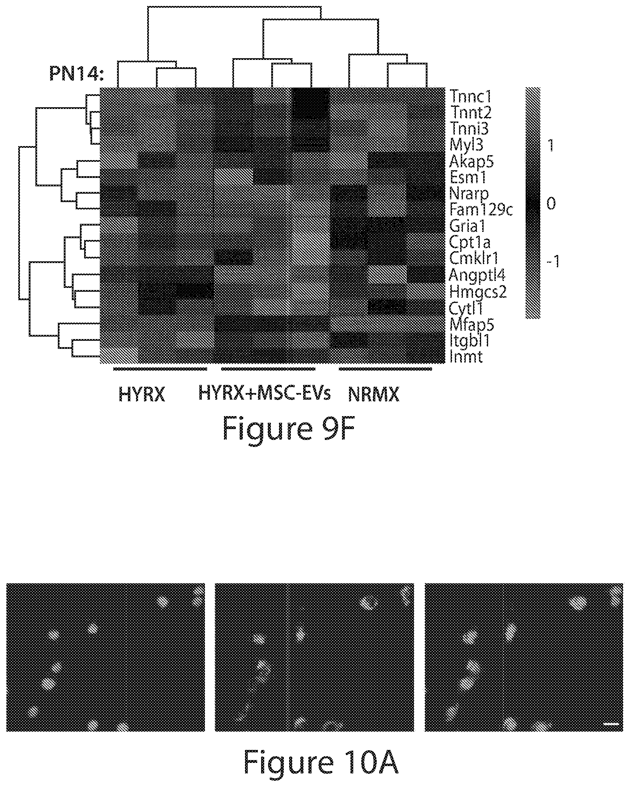

**Figures 9A to 9F****. Global analysis of mRNA-sequencing data from mouse lungs at PN7 and PN14. (****Figure 9A****)** Principle component analysis (PCA) of gene expression profiles obtained from RNA extracted from mouse lungs at PN7 or PN14. **(****Figure 9B****)** MA plots between NRMX mice versus HYRX-exposed mice, and NRMX versus HYRX-exposed mice treated with MSC-EVs for PN7 and PN14. An MA plot displays the log-fold change in mRNA expression compared to the mean expression (normalized counts). DESeq2 was used to determine differentially expressed transcripts (padj <0.1), which are marked with red. **(****Figure 9C****)** The top 10 categories from gene ontology (GO) enrichment analysis (biological process) of the genes (at PN7) that were uniquely up-regulated by MSC-EV treatment compared to HYRX and NRMX controls. **(****Figure 9D****)** Genes that were elevated in the HYRX-group compared to NRMX mice. **(****Figure 9E****)** The top 10 categories from GO enrichment analysis (biological process) of the genes that were significantly suppressed by the MSC-EV treatment compared to HYRX-control mice. **(****Figure 9F****)** Heat map highlighting genes that are significantly modulated by MSC-EV treatment at PN14.

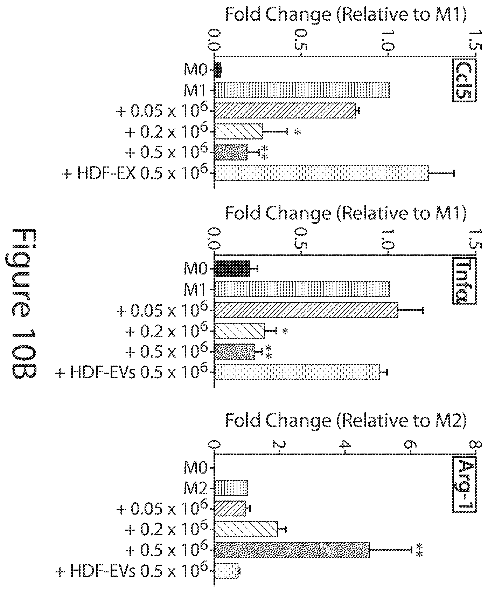

**Figures 10A to 10B****. Alveolar macrophage-WJMSC-EV interaction. (****Figure 10A****)** Using fluorescence microscopy it was observed that WJMSC-EVs readily interacted with alveolar macrophages (MH-S cells). Scale bar = 20 µm. **(****Figure 10B****)** In a dose dependent manner, the addition of WJMSC-EVs to MH-S cells reduced the mRNA induction of classically-activated (M1) macrophage markers such as Ccl5 and TNFα, whereas the addition of WJMSC-EVs to an alternatively polarized (M2) macrophage dose dependently augmented the induction of Arg-1 mRNA. + 0.05-0.5 × 10⁶ refers to the MSC-EV (cell equivalent) dose. Mean ± SEM, n = 3-5 per group, * p < 0.05, ** p < 0.01.

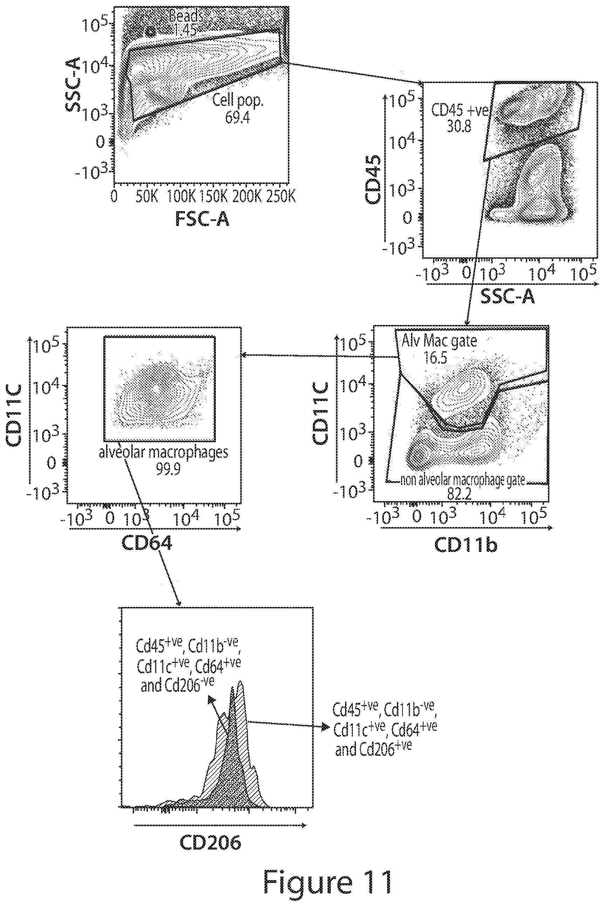

**Figure 11****. Representative *in vivo* lung macrophage gating strategy.** Representative gating strategy used to identify lung macrophage cell populations in the mouse lung. Cells were isolated from enzymatically digested mouse lungs. Excluding cell aggregates and cell debris (based on forward and side scatter parameters), immune cells were identified by Cd45 staining. A sequential gating strategy was used to identify lung macrophage populations expressing specific markers (Cd45^{+ve}, Cd11b^{-ve}, Cd11c^{+ve}, Cd64^{+ve}). Following lung macrophage identification, positive macrophage phenotype makers (Cd40, Cd86 and Cd206 (chosen above) were assessed by comparing to appropriate fluorescence-minus-one controls. Left histogram: (Cd45^{+ve}, Cd11b^{-ve}, Cd11c^{+ve}, Cd64^{+ve} and Cd206^{-ve}). Right histogram: (Cd45^{+ve}, Cd 11b^{-ve}, Cd11c^{+ve}, Cd64^{+ve} and Cd206^{+ve}).

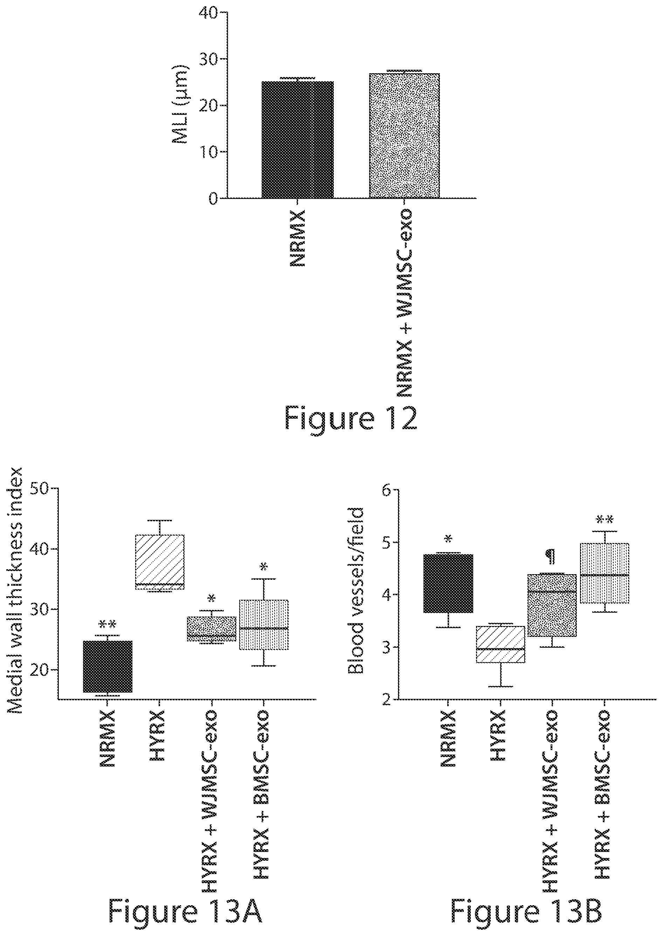

**Figure 12****. WJMSC-exo administration has no effect on non-injured** lung. NRMX-control animals received a single intravenous (IV) dose of WJMSC-exos at PN4 (as described in the Methods). On assessment of lung architecture at PN14, no difference was found between NRMX-control mice and NRMX-mice treated with WJMSC-exos (p = 0.9). MLI: Mean linear intercept.

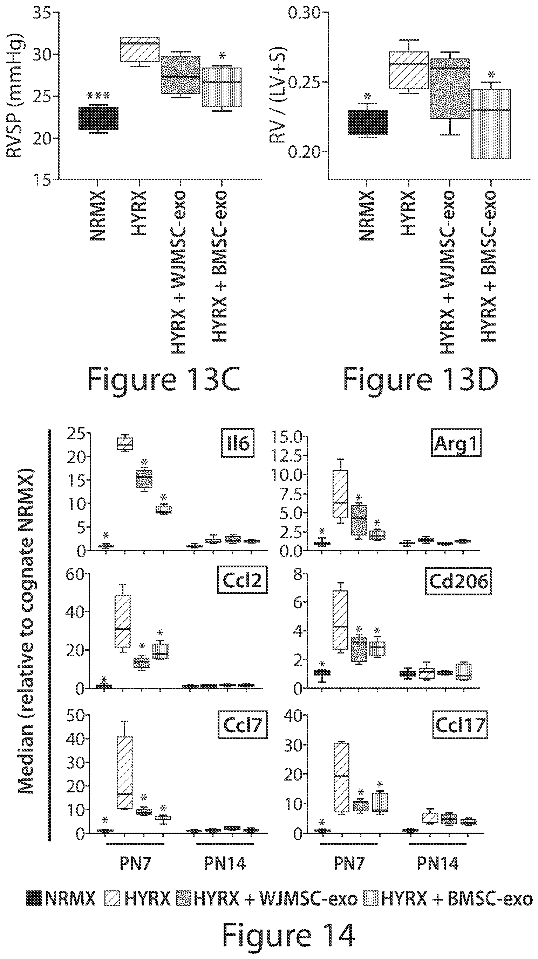

**Figures 13A-13D** WJMSC-exo and BMSC-exo treatment demonstrate similar trends in ameliorating pulmonary hypertension, preventing hyperoxia-induced loss of microvasculature, rescues peripheral pulmonary arterial remodeling. **(****Figure 13A****)** To assess the potential impact of MSC-exo treatments on HYRX-induced peripheral pulmonary vascular remodeling, lung sections of mice harvested at PN42 were stained with α-smooth muscle actin (α-SMA). Medial thickness index = 100 × (area[ext] - area[int]) / area[ext] ). Area[ext] and area[int] denote the areas within the external and internal boundaries of the α-SMA layer, respectively. Medial wall thickness values are shown for the two MSC-exo groups (WJMSC-exo and BMSC-exo) compared with NRMX and HYRX controls. n = 4-5 per group. **(****Figure 13B****)** MSC-exos prevent hyperoxia-induced loss of blood vessels in the peripheral microvasculature. Lung sections were stained for von Willebrand factor (vWF) in mice harvested at PN42. n = 5 per group. To quantify the degree of pulmonary hypertension, we undertook direct RVSP measurements **(****Figure 13C****)** and assessed Fulton's index **(****Figure 13D** **)** in mice at PN42. n = 6-12 per group. Mean ± SEM. *p <0.05, **p <0.01, ***p <0.001, ¶p= 0.06 vs. HYRX.
**Figure 14****. WJMSC-exo and BMSC-exo alter the lung transcriptome.** Validation of select whole-lung RNA-seq data and suppression of candidate proinflammatory markers by WJMSC-exo and BMSC-exo treatment was assessed by RT-qPCR in separate experiments. Mean ± SEM, n = 3-4 per group, * p < 0.05 vs. HYRX controls.

### DETAILED DESCRIPTION OF CERTAIN EMBODIMENTS

The scope of the invention is defined by the claims. Any reference in the description to methods of treatment refer to the products of the present invention for use in a method for treatment.

The present disclosure is based, at least in part, on the surprising finding that mesenchymal stem cell exosomes (MSC exosomes) are heterogeneous and a subpopulation of the total MSC exosomes afford the therapeutic efficacy of MSC exosomes in treating lung diseases, e.g., bronchopulmonary dysplasia (BPD). The purified fraction of MSC exosomes are high in exosome contents and low in protein contaminants. Protein markers that are differentially represented on the purified fraction of MSC exosomes, compared to other fractions of MSC exosomes or exomes derived from other cell types, are identified. The purified MSC exosomes are found to possess immunomodulatory activities and are effective in treating various disorders (e.g., lung diseases). Methods of purifying the subpopulation of exosomes are also provided.

Accordingly, some aspects of the present disclosure relate to isolated MSC exosomes. An "exosome" is a membrane (e.g., lipid bilayer) vesicle that is released from a cell (e.g., any eukaryotic cell). Exosomes are present in eukaryotic fluids, including blood, urine, and cultured medium of cell cultures. The exosomes of the present disclosure are released from mesenchymal stem cells (MSCs) and are interchangeably termed "mesenchymal stem cell exosomes" or "MSC exosomes." Similarly, exosomes released from a fibroblast are termed "fibroblast exosomes" herein. It is surprisingly found herein that exosomes released from other cell types, e.g., fibroblasts, do not have the same properties (e.g., protein markers and biological activities) as the MSC exosomes.

A "mesenchymal stem cell (MSC)" is a progenitor cell having the capacity to differentiate into neuronal cells, adipocytes, chondrocytes, osteoblasts, myocytes, cardiac tissue, and other endothelial or epithelial cells. (See for example Wang, Stem Cells 2004;22(7);1330-7; McElreavey;1991 Biochem Soc Trans (1);29s; Takechi, Placenta 1993 March/April; 14 (2); 235-45; Takechi, 1993; Kobayashi; Early Human Development;1998; July 10; 51 (3); 223-33; Yen; Stem Cells; 2005; 23 (1) 3-9.) These cells may be defined phenotypically by gene or protein expression. These cells have been characterized to express (and thus be positive for) one or more of CD13, CD29, CD44, CD49a, b, c, e, f, CD51, CD54, CD58, CD71, CD73, CD90, CD102, CD105, CD106, CDw119, CD120a, CD120b, CD123, CD124, CD126, CD127, CD140a, CD166, P75, TGF-bIR, TGF-bIIR, HLA-A, B, C, SSEA-3, SSEA-4, D7 and PD-L1. These cells have also been characterized as not expressing (and thus being negative for) CD3, CD5, CD6, CD9, CD10, CD11a, CD14, CD15, CD18, CD21, CD25, CD31, CD34, CD36, CD38, CD45, CD49d, CD50, CD62E, L, S, CD80, CD86, CD95, CD117, CD133, SSEA-1, and ABO. Thus, MSCs may be characterized phenotypically and/or functionally according to their differentiation potential.

MSCs may be harvested from a number of sources including but not limited to bone marrow, blood, periosteum, dermis, umbilical cord blood and/or matrix (e.g., Wharton's Jelly), and placenta. Methods for harvesting MSCs are described in the art, e.g., in US Patent No. 5486359,

MSCs can be isolated from multiple sources, e.g., bone marrow mononuclear cells, umbilical cord blood, adipose tissue, placental tissue, based on their adherence to tissue culture plastic. For example, MSCs can be isolated from commercially available bone marrow aspirates. Enrichment of MSCs within a population of cells can be achieved using methods known in the art including but not limited to fluorescence-activated cell sorting (FACS).

Commercially available media may be used for the growth, culture and maintenance of MSCs. Such media include but are not limited to Dulbecco's modified Eagle's medium (DMEM). Components in such media that are useful for the growth, culture and maintenance of MSCs, fibroblasts, and macrophages include but are not limited to amino acids, vitamins, a carbon source (natural and non-natural), salts, sugars, plant derived hydrolysates, sodium pyruvate, surfactants, ammonia, lipids, hormones or growth factors, buffers, non-natural amino acids, sugar precursors, indicators, nucleosides and/or nucleotides, butyrate or organics, DMSO, animal derived products, gene inducers, non-natural sugars, regulators of intracellular pH, betaine or osmoprotectant, trace elements, minerals, non-natural vitamins. Additional components that can be used to supplement a commercially available tissue culture medium include, for example, animal serum (e.g., fetal bovine serum (FBS), fetal calf serum (FCS), horse serum (HS)), antibiotics (e.g., including but not limited to, penicillin, streptomycin, neomycin sulfate, amphotericin B, blasticidin, chloramphenicol, amoxicillin, bacitracin, bleomycin, cephalosporin, chlortetracycline, zeocin, and puromycin), and glutamine (e.g., L-glutamine). Mesenchymal stem cell survival and growth also depends on the maintenance of an appropriate aerobic environment, pH, and temperature. MSCs can be maintained using methods known in the art, e.g., as described in Pittenger et al., Science, 284:143-147 (1999).

A fibroblast is a type of cell that synthesizes the extracellular matrix and collagen, the structural framework (e.g., stroma) for animal tissues, and plays a critical role in wound healing. Fibroblasts are the most common cells of connective tissue in animals. Fibroblasts typically have a branched cytoplasm surrounding an elliptical, speckled nucleus having two or more nucleoli. Active fibroblasts can be recognized by their abundant rough endoplasmic reticulum. Inactive fibroblasts, which are also called fibrocytes, are smaller and spindle shaped. They have a reduced rough endoplasmic reticulum.

Sources of fibroblasts include connective tissues such as loose, dense, elastic, reticular, and adipose connective tissue. In addition, there are embryonic connective tissues, as well as specialized connective tissues, which include bone, cartilage, and blood. Other sources include the skin. Methods for isolating and culturing fibroblasts are well known in the art (See, e.g., Weber et al., "Isolation and Culture of Fibroblasts, Vascular Smooth Muscle, and Endothelial Cells From the Fetal Rat Ductus Arteriosus." Pediatric Research. 2011; 70, 236-241; Huschtscha et al., "Enhanced isolation of fibroblasts from human skin explants." Biotechniques. 2012;53(4):239-44.

Some aspects of the present disclosure relate to isolated exosomes. As used herein, an "isolated exosome" is an exosome that is physically separated from its natural environment. An isolated exosome may be physically separated, in whole or in part, from tissue or cells with which it naturally exists, including MSCs, fibroblasts, and macrophages. In the present invention
, the isolated exosomes are isolated MSC exosomes, In some embodiments, the isolated MSC exosomes are isolated from the culturing media of MSCs from human bone marrow, or umbilical cord Wharton's Jelly. Such culturing media is termed "MSC-conditioned media" herein. In some embodiments, isolated exosomes may be free of cells such as MSCs, or it may be free or substantially free of conditioned media, or it may be free of any biological contaminants such as proteins. Typically, the isolated exosomes are provided at a higher concentration than exosomes present in unmanipulated conditioned media.

The isolated MSC exosomes of the present disclosure are characterized for markers (e.g., proteins) that are present or enriched compared to exosomes derived from other cell types (e.g., a fibroblast), or in other fractions resulting from the purification process. A "marker," as used herein, refers to a molecule (e.g., a protein) that is associated with the MSC exosome and co-purifies with the MSC exosome in the purification methods described herein. A marker that is associated with the MSC exosome may bind to the lipid membrane of the MSC exosome (e.g., binds on the outer surface, or is inserted into the lipid membrane), or be encapsulated by the MSC exosome. Methods of detecting certain markers in an exosome is known to those skilled in the art, e.g., western blotting, immunostaining, or mass spectrometry.

In some embodiments, the isolated MSC exosomes of the present disclosure comprises four or more (e.g., 4, 5, 6, 7, 8, 9, 10, 11, 12, 13, 14, 15, 16, 17, 18, 19, 20, or 21) markers selected from the group consisting of: FLT1 (Vascular endothelial growth factor receptor 1, Uniprot ID P17948), PLAUR (Urokinase plasminogen activator surface receptor, Uniprot ID Q03405), MME (Neprilysin, Uniprot ID P08473), CDH2 (Cadherin-2, P19022), SLC16A3 (Monocarboxylate transporter 4, Uniprot ID: 015427), CDH13 (Cadherin-13, Uniprot ID: P55290), ITGB5 (Integrin beta-5, Uniprot ID: P18084), ITGA11 (Integrin alpha-11, Uniprot ID: Q9UKX5), APP (Amyloid beta A4 protein, Uniprot ID: P05067), GPC6 (Glypican-6, Uniprot ID: Q9Y625), IGSF8 (Immunoglobulin superfamily member 8, Uniprot ID: Q969P0), IRP2 (Iron-responsive element-binding protein 2, also known as IREB2, uniprot ID: P48200), TSPAN9 (Tetraspanin-9, Uniprot ID: 075954), DAG1 (Dystroglycan, Uniprot ID: Q14118), SLC38A2 (Sodium-coupled neutral amino acid transporter 2, Uniprot ID: Q96QD8), SLC12A8 (Solute carrier family 12 member 8, Uniprot ID: AOAV02), GJA1 (Gap junction alpha-1 protein, Uniprot ID: P17302), ITGA1 (Integrin alpha-1, Uniprot ID: P56199), PLXNB2 (Plexin-B2, Uniprot ID: 015031), SLC16A1 (Monocarboxylate transporter 1, Umiprot ID: P53985), and PROCR (Endothelial protein C receptor, Uniprot ID: Q9UNN8). The isolated MSC exosome "comprises a marker" means that the marker is detectable in the isolated exosome, e.g., by western blotting, immunostaining, or mass spectrometry. In some embodiments, the marker is not present (e.g., not detectable) in exosomes derived from cell types other than MSCs, e.g., fibroblasts.

In some embodiments, the isolated MSC exosomes of the present disclosure comprises one or more (e.g., 1, 2, 3, 4, 5, 6, 7, 8, 9, 10, 11, or 12) markers that is enriched compared to a fibroblast exosome, the one or more markers selected from the group consisting of: PTk7 (Inactive tyrosine-protein kinase 7, Uniprot ID: Q13308), CD109 (CD109 antigen, Uniprot ID: Q6YHK3), SLC1A5 (Neutral amino acid transporter B(0), Uniprot ID: Q15758), ITGA3 (Integrin alpha-3, Uniprot ID: P26006), ITGA2 (Integrin alpha-2, Uniprot ID: P17301), BSG (Basigin, Uniprot ID: P35613), DPP4 (Dipeptidyl peptidase 4, Uniprot ID: P27487), ATP1A1 (Sodium/potassium-transporting ATPase subunit alpha-1, Uniprot ID: P05023), FAP (Prolyl endopeptidase FAP, Uniprot ID: Q12884), VASN (Vasorin, Uniprot ID: Q6EMK4), IGF2R (Cation-independent mannose-6-phosphate receptor, Uniprot ID: P11717), and CD82 (CD82 antigen, Uniprot ID: P27701).

In some embodiments, the isolated MSC exosomes of the present disclosure comprises one or more (e.g., 1, 2, 3, 4, 5, 6, 7, 8, 9, 10, 11, 12, 13, 14, 15, 16, 17, 18, 19, 20, 21, 22, 23, or 24) markers that is enriched compared to a fibroblast exosome, the one or more markers selected from the group consisting of: RCN1(Reticulocalbin-1, Uniprot ID: Q15293), RAP1B (Ras-related protein Rap-1b, Uniprot ID: P61224), GDF15 (Growth/differentiation factor 15, Uniprot ID: Q99988), FLT1 (Vascular endothelial growth factor receptor 1, Uniprot ID: P17948), OLFML3 (Olfactomedin-like protein 3, Uniprot ID: Q9NRN5), PLOD2 (Procollagen-lysine,2-oxoglutarate 5-dioxygenase 2, Uniprot ID: O00469), PSMA4 (Proteasome subunit alpha type-4, Uniprot ID: P25789), PAPPA (Pappalysin-1, Uniprot ID: Q13219), INHBA (Inhibin beta A chain, Uniprot ID: P08476), SPOCK1 (Testican-1, Uniprot ID: Q08629), HSPB1 (Heat shock protein beta-1, Uniprot ID: P04792), LOXL4 (Lysyl oxidase homolog 4, Uniprot ID: Q96JB6), POLD3 (DNA polymerase delta subunit 3, Uniprot ID: Q15054), CALU (Calumenin, Uniprot ID: O43852), IGFBP4 (Insulin-like growth factor-binding protein 4, Uniprot ID: P22692), C4B (Complement C4-B, Uniprot ID: P0C0L5), TINAGL1 (Tubulointerstitial nephritis antigen-like, Uniprot ID: Q9GZM7), SULF1 (Extracellular sulfatase Sulf-1, Uniprot ID: Q8IWU6), TPM3 (Tropomyosin alpha-3 chain, Uniprot ID: P06753), AHNAK (Neuroblast differentiation-associated protein AHNAK, Uniprot ID: Q09666), CALR (Calreticulin, Uniprot ID: P27797), RRAS2 (Ras-related protein R-Ras2, Uniprot ID: P62070), PFKP (ATP-dependent 6-phosphofructokinase, platelet type, Uniprot ID: Q01813), and COL16A1 (Collagen alpha-1(XVI) chain, Uniprot ID: Q07092).

A marker being "enriched" in the isolated MSC exosomes, means that the marker is present in the isolated MSC exosome at a level that is at least 20%, at least 30%, at least 40%, at least 50%, at least 60%, at least 70%, at least 80%, at least 90%, at least 100%, at least 2-fold, at least 5-fold, at least 10-fold, at least 20-fold, at least 30-fold, at least 40-fold, at least 50-fold, at least 60-fold, at least 70-fold, at least 80-fold, at least 90-fold, at least 100-fold or more, higher than the level of the marker in exosomes derived from other cell types, e.g., a fibroblast. In some embodiments, the marker is present in the isolated MSC exosome at a level that is 20%, 30%, 40%, 50%, 60%, 70%, 80%, 90%, 100%, 2-fold, 5-fold, 10-fold, 20-fold, 30-fold, 40-fold, 50-fold, 60-fold, 70-fold, 80-fold, 90-fold, 100-fold or more, higher than the level of the marker in exosomes derived from other cell types, e.g., a fibroblast exosome.

In some embodiments, the isolated MSC exosome described herein comprises one or more (e.g., 1, 2, 3, 4, 5, or more) known exosome markers. In some embodiments, the known exosome markers are selected from the group consisting of: FLOT1 (Flotillin-1, Uniprot ID: O75955), CD9 (CD9 antigen, Uniprot ID: P21926), and CD63 (CD63 antigen, Uniprot ID: P08962).

In some embodiments, the isolated MSC exosome is substantially free of contaminants (e.g., protein contaminants). The isolated MSC exosome is "substantially free of contaminants" when the preparation of the isolated MSC exosome contains fewer than 20%, 15%, 10%, 5%, 2%, 1%, or less than 1%, of any other substances (e.g., proteins). In some embodiments, the isolated MSC is "substantially free of contaminants" when the prepare of the isolated MSC exosome is at least 80%, at least 85%, at least 90%, at least 95%, at least 98%, at least 99%, at least 99.9% pure, with respect to contaminants (e.g., proteins).

"Protein contaminants" refer to proteins that are not associated with the isolated exosome and do not contribute to the biological activity of the exosome. The protein contaminants are also referred to herein as "non-exosomal protein contaminants."

The isolated MSC exosome described herein may have a diameter of about 30-150 nm. For example, the isolated MSC exosome may have a diameter of 30-150, 30-140, 30-130, 30-120, 30-110, 30-100, 30-90, 30-80, 30-70, 30-60, 30-50, 30-40, 40-150, 40-140, 40-130, 40-120, 40-110, 40-100, 40-90, 40-80, 40-70, 40-60, 40-50, 50-150 nm, 50-140 nm, 50-130 nm, 50-120 nm, 50-110 nm, 50-100 nm, 50-90 nm, 50-80 nm, 50-70 nm, 50-60 nm, 60-150 nm, 60-140 nm, 60-130 nm, 60-120 nm, 60-110 nm, 60-100 nm, 60-90 nm, 60-80 nm, 60-70 nm, 70-150 nm, 70-140 nm, 70-130 nm, 70-120 nm, 70-110 nm, 70-100 nm, 70-90 nm, 70-80 nm, 80-150 nm, 80-140 nm, 80-130 nm, 80-120 nm, 80-110 nm, 80-100 nm, 80-90 nm, 90-150 nm, 90-140 nm, 90-130 nm, 90-120 nm, 90-110 nm, 90-100 nm, 100-150 nm, 100-140 nm, 100-130 nm, 100-120 nm, 100-110 nm, 110-150 nm, 110-140 nm, 110-130 nm, 110-120 nm, 120-150 nm, 120-140 nm, 120-130 nm, 130-150 nm, 130-140 nm, or 140-150 nm. In some embodiments, the isolated MSC exosome may have a diameter of about 50 nm, 60 nm, 70 nm, 80 nm, 90 nm, 100 nm, 110 nm, 120 nm, 130 nm, 140 nm, or 150 nm. In some embodiments, the isolated MSC exosomes exhibit a biconcave morphology.

As described herein, the isolated MSC exosome is biologically active and the therapeutic efficacy of MSC exosomes in treating lung diseases may be attributed to this isolated (purified) fraction. As such, the isolated MSC exosomes may be used in treating diseases (e.g., a lung disease), or in the manufacturing of a medicament that treats diseases (e.g., lung diseases).

Some aspects of the present disclosure relate to pharmaceutical compositions comprising the isolated MSC exosomes described herein. In some embodiments, such pharmaceutical compositions further comprise pharmaceutically acceptable concentrations of salt, buffering agents, preservatives, compatible carriers, and and/or other (i.e., secondary) therapeutic agents.

A pharmaceutically acceptable carrier is a pharmaceutically acceptable material, composition or vehicle, such as a liquid or solid filler, diluent, excipient, solvent or encapsulating material, involved in carrying or transporting a prophylactically or therapeutically active agent. Each carrier must be "acceptable" in the sense of being compatible with the other ingredients of the formulation and not injurious to the subject. Some examples of materials which can serve as pharmaceutically acceptable carriers include sugars, such as lactose, glucose and sucrose; glycols, such as propylene glycol; polyols, such as glycerin, sorbitol, mannitol and polyethylene glycol; esters, such as ethyl oleate and ethyl laurate; buffering agents, such as magnesium hydroxide and aluminum hydroxide; pyrogen-free water; isotonic saline; Ringer's solution; ethyl alcohol; phosphate buffer solutions; and other non-toxic compatible substances employed in pharmaceutical formulations.

In some embodiments, the pharmaceutical composition comprising the isolated MSC exosomes is formulated for the treatment of lung diseases. As such, the pharmaceutical composition may further comprise other therapeutic agents (secondary agents) that may be used to treatment lung diseases. As used herein, a therapeutic agent refers to any agent which can be used in the prevention, treatment and/or management of a lung disease such as those discussed herein. These include but are not limited to surfactants, inhaled nitric oxide, almitrine bismesylate, immunomodulators, and antioxidants. Examples of immunomodulators include steroids and corticosteroids such as but not limited to methylprednisolone. Examples of antioxidants include but are not limited to superoxide dismutase.

Certain secondary therapeutic agents used in the treatment or management of certain lung and other diseases including but not limited to pulmonary hypertension include oxygen, anticoagulants such as warfarin (Coumadin); diuretics such as furosemide (Lasix^{®}) or spironalactone (Aldactone^{®}); calcium channel blockers; potassium such as K-dur^{®}; inotropic agents such as digoxin; vasodilators such as nifedipine (Procardia^{®}) or diltiazem (Cardizem^{®}); endothelin receptor antagonists such as bosentan (Tracleer^{®}) and ambrisentan (Letairis^{®}); prostacyclin analogues such as epoprostenol (Flolan^{®}), treprostinil sodium (Remodulin^{®}, Tyvaso^{®}), and iloprost (Ventavis^{®}); and PDE-5 inhibitors such as sildenafil (Revatio^{®}) and tadalafil (Adcirca^{®}).

In some embodiments, for the treatment of lung diseases, the second agent is a pulmonary surfactant. A "pulmonary surfactant" is a lipoprotein mixture useful in keeping lung airways open (e.g., by preventing adhesion of alveolar walls to each other). Pulmonary surfactants may be comprised of phospholipids such as dipalmitoylphosphatidylcholine (DPPC), phosphotidylcholine (PC), phosphotidylglycerol (PG); cholesterol; and proteins such as SP-A, B, C and D. Pulmonary surfactants may be derived from naturally occurring sources such as bovine or porcine lung tissue. Examples include Alveofact^{™} (from cow lung lavage), Curosurf^{™} (from minced pig lung), Infasurf^{™} (from calf lung lavage), and Survanta^{™} (from minced cow lung, with additional components including DPPC, palmitic acid, and tripalmitin). Pulmonary surfactants may also be synthetic. Examples include Exosurf^{™} (comprised of DPPC with hexadecanol and tyloxapol), Pumactant^{™} or Artificial Lung Expanding Compound (ALEC) (comprised of DPPC and PG), KL-4 (comprised of DPPC, palmitoyl-oleoyl phosphatidylglyercol, palmitic acid, and synthetic peptide that mimics SP-B), Venticute^{™} (comprised of DPPC, PG, palmitic acid, and recombinant SP-C). Pulmonary surfactants may be obtained from commercial suppliers.

Other aspects of the present disclosure provide
a pharmaceutical composition according to the present invention, for use in treating hyperoxia-induced lung injury, hyperoxia-induced pulmonary hypertension, bronchopulmonary dysplasia, and reducing pulmonary fibrosis in a subject.

Lung diseases that may be treated
include, without limitation, pulmonary hypertension (PH) which is also referred to as pulmonary artery hypertension (PAH), asthma, bronchopulmonary dysplasia (BPD), allergies, sarcoidosis, and idiopathic pulmonary fibrosis. These diseases also include lung vascular diseases which may not have an inflammatory component. Still other pulmonary conditions that may be treated include acute lung injury which may be associated with sepsis or with ventilation. An example of this latter condition is acute respiratory distress syndrome which occurs in older children and adults.

The pharmaceutical composition described herein may be used to treat bronchopulmonary dysplasia (BPD). BPD is a condition that affects neonates who have been given oxygen or have been on ventilators, or neonates born prematurely particularly those born very prematurely (e.g., those born before 32 weeks of gestation). It is also referred to as neonatal chronic lung disease. Causes of BPD include mechanical injury, for example as a result of ventilation, oxygen toxicity, for example as a result of oxygen therapy, and infection. The disease may progress from non-inflammatory to inflammatory with time. Symptoms include bluish skin, chronic cough, rapid breathing, and shortness of breath. Subjects having BPD are more susceptible to infections such as respiratory syncytial virus infection. Subjects having BPD may develop pulmonary hypertension. Subjects have BPD may also have inflammation in the lungs, and exhibit peripheral pulmonary arterial remodeling and pulmonary fibrosis. The present disclosure describes methods of treating bronchopulmonary dysplasia (BPD) in a subject. As demonstrated herein, a bolus dose of the isolated MSC exosome is effective in ameliorating the short-term and long-term symptoms associated with BPD. The subject that may be treated using the methods described herein may be a neonate, or a child, or an adult that is suffering from or has suffered from BPD.

The pharmaceutical composition described herein may be used to treat pulmonary hypertension. Pulmonary hypertension is a lung disease characterized by blood pressure in the pulmonary artery that is above normal levels. Symptoms include shortness of breath, chest pain particularly during physical activity, weakness, fatigue, fainting, light headedness particularly during exercise, dizziness, abnormal heart sounds and murmurs, engorgement of the jugular vein, retention of fluid in the abdomen, legs and ankles, and bluish coloring in the nail bed. In some embodiments, hyperoxia condition (e.g., when a subject is administered oxygen) can induce pulmonary hypertention (hyperoxia-induced pulmonary hypertention).

The pharmaceutical composition described herein may be used to treat acute respiratory distress syndrome (ARDS). ARDS is also known as respiratory distress syndrome (RDS) or adult respiratory distress syndrome is a condition that arises as a result of injury to the lungs or acute illness. The injury to the lung may be a result of ventilation, trauma, burns, and/or aspiration. The acute illness may be infectious pneumonia or sepsis. It is considered a severe form of acute lung injury, and it is often fatal. It is characterized by lung inflammation, impaired gas exchange, and release of inflammatory mediators, hypoxemia, and multiple organ failure. ARDS can also be defined as the ratio of arterial partial oxygen tension (PaO2) as a fraction of inspired oxygen (FiO2) below 200 mmHg in the presence of bilateral infiltrates on the chest x-ray. A PaO2/FiO2 ratio less than 300 mmHg with bilateral infiltrates indicates acute lung injury, which is often a precursor to ARDS. Symptoms of ARDS include shortness of breath, tachypnea, and mental confusion due to low oxygen levels.

The pharmaceutical composition described herein may be used to treat idiopathic pulmonary fibrosis. Idiopathic pulmonary fibrosis is characterized by scarring or thickening of the lungs without a known cause. It occurs most often in persons 50-70 years of age. Its symptoms include shortness of breath, regular cough (typically a dry cough), chest pain, and decreased activity level.

The pharmaceutical composition described herein may be used to treat allergy. Allergy is a hypersensitivity disorder of the immune system, with symptoms including red eyes, itchiness, and runny nose, eczema, hives, or an asthma attack. Allergies play a major role in conditions such as asthma. Severe allergies to environmental or dietary allergens or to medication may result in life-threatening reactions called anaphylaxis. Allergic reactions can occur when a person's immune system reacts to what is often a normally harmless substance in the environment. Allergy is one of four forms of hypersensitivity and is sometimes called type I (or immediate) hypersensitivity. Allergic reactions are distinctive because of excessive activation of certain white blood cells (mast cells and basophils) by Immunoglobulin E (IgE). This reaction results in an inflammatory response which can range from mild discomfort to dangerous. A variety of tests exist to diagnose allergic conditions. Tests include placing possible allergens on the skin and looking for a reaction such as swelling and blood tests to look for an allergen-specific IgE.

The pharmaceutical composition described herein may be used to treat hypoxia-induced lung inflammation. Hypoxia-induced lung inflammation is a condition often resulting from acute lung injury and/or ARDS, whereby an inflammatory response results from prolonged exposure to hypoxic conditions. Such inflammatory response includes increased macrophages, neutrophils, and inflammatory cytokines, including IL-1β, IL-6, IL-8, and TNF-α, in the bronchoalveolar lavage fluid of humans exposed to hypobaric hypoxia.

An "effective amount" is the amount of an agent that achieves the desired outcome. The absolute amount will depend upon a variety of factors, including the material selected for administration, whether the administration is in single or multiple doses, and individual patient parameters including age, physical condition, size, weight, and the stage of the disease. These factors are well known to those of ordinary skill in the art and can be addressed with no more than routine experimentation.

The effective amount may be the dosage of an agent that causes no toxicity to the subject. The effective amount may be the dosage of an agent that causes reduced toxicity to the subject. Methods for measuring toxicity are well known in the art (e.g., biopsy/histology of the liver, spleen, and/or kidney; alanine transferase, alkaline phosphatase and bilirubin assays for liver toxicity; and creatinine levels for kidney toxicity).

"Treat" or "treatment" includes, but is not limited to, preventing, reducing, or halting the development of a lung disease, reducing or eliminating the symptoms of lung disease, or preventing lung disease.

A subject shall mean a human or vertebrate animal or mammal including but not limited to a rodent, e.g., a rodent such as a rat or a mouse, dog, cat, horse, cow, pig, sheep, goat, turkey, chicken, and primate, e.g., monkey. The methods of the present disclosure are useful for treating a subject in need thereof. A subject in need thereof can be a subject who has a risk of developing a lung disease (i.e., via a genetic test) or a subject who has lung disease.

The subjects may be those that have a disease (or condition) described herein amenable to treatment using the exosomes described in this disclosure, or they may be those that are at risk of developing such a disease (or condition). Such subjects include neonates and particularly neonates born at low gestational age. As used herein, a human neonate refers to an human from the time of birth to about 4 weeks of age. As used herein, a human infant refers to a human from about the age of 4 weeks of age to about 3 years of age. As used herein, low gestational age refers to birth (or delivery) that occurs before a normal gestational term for a given species. In humans, a full gestational term is about 40 weeks and may range from 37 weeks to more than 40 weeks. Low gestational age, in humans, akin to a premature birth is defined as birth that occurs before 37 weeks of gestation. The disclosure therefore contemplates prevention and/or treatment of subjects born before 37 weeks of gestation, including those born at even shorter gestational terms (e.g., before 36, before 35, before 34, before 33, before 32, before 31, before 30, before 29, before 28, before 27, before 26, or before 25 weeks of gestation). In some embodiments, the subject has been administered oxygen. In some embodiments, the subject has been subjected to mechanical interventions such as ventilation with or without exogenous oxygen administration. In some embodiments, the subject has hyperoxia-induced lung injury. In some embodiments, the subject has developed or is at risk of developing BPD.

For infants or neonates, the present disclosure contemplates their treatment even beyond the neonate stage and into childhood and/or adulthood. For example, in some embodiments, the subject treated is 3-18 years of age. In some embodiments, the subject treated
may be 3-18, 3-17, 3-16, 3-15, 3-14, 3-13, 3-12, 3-11, 3-10, 3-9, 3-8, 3-7, 3-6, 3-5, 3-4, 4-18, 4-17, 4-16, 4-15, 4-14, 4-13, 4-12, 4-11, 4-10, 4-9, 4-8, 4-7, 4-6, 4-5, 5-18, 5-17, 5-16, 5-15, 5-14, 5-13, 5-12, 5-11, 5-10, 5-9, 5-8, 5-7, 5-6, 6-18, 6-17, 6-16, 6-15, 6-14, 6-13, 6-12, 6-11, 6-10, 6-9, 6-8, 6-7, 7-18, 7-17, 7-16, 7-15, 7-14, 7-13, 7-12, 7-11, 7-10, 7-9, 7-8, 8-18, 8-17, 8-16, 8-15, 8-14, 8-13, 8-12, 8-11, 8-10, 8-9, 9-18, 9-17, 9-16, 9-15, 9-14, 9-13, 9-12, 9-11, 9-10, 10-18, 10-17, 10-16, 10-15, 10-14, 10-13, 10-12, 10-11, 11-18, 11-17, 11-16, 11-15, 11-14, 11-13, 11-12, 12-18, 12-17, 12-16, 12-15, 12-14, 12-13, 13-18, 13-17, 13-16, 13-15, 13-14, 14-18, 14-17, 14-16, 14-15, 15-18, 15-17, 15-16, 16-18, 16-17, or 17-18 years of age. In some embodiments, the subject is an adult, e.g., 18 or more than 18 years of age.

Certain subjects may have a genetic predisposition to certain forms of the diseases (or conditions) described herein such as for example pulmonary hypertension, and those subjects may also be treated according to the disclosure.

With respect to neonates and particularly low gestation age neonates, the disclosure contemplates administration of the isolated MSC exosomes within 1 year, 11 months, 10 months, 9 months, 8 months, 7 months, 6 months, 5 months, 4 months, 3 months, 2 months, 1 month, 4 weeks, 3 weeks, 2 weeks, 1 week, 6 days, 5 days, 4 days, 3 days, 2 days, 1 day, 12 hours, 6 hours, 3 hours, or 1 hour of birth. In some embodiments, the isolated MSC exosomes are administered within 1 hour of birth (e.g., within 1 hour, within 55 minutes, within 50 minutes, within 45 minutes, within 40 minutes, within 35 minutes, within 30 minutes, within 25 minutes, within 20 minutes, within 15 minutes, within 10 minutes, within 5 minutes, or within 1 minute).

The present disclosure further contemplates administration of the isolated MSC exosomes even in the absence of symptoms indicative of a disease or disorder as described herein.

In some embodiments, the isolated MSC exosome or the composition comprising the isolated exosome is administered to a subject (e.g., a neonate) in a bolus dose. A "bolus dose" refers to the single administration of a discrete amount of medication, drug or other compound in order to achieved a therapeutically effective level. In some embodiments, repeated administration of the isolated MSC exosomes, including two, three, four, five or more administrations of the isolated MSC exosomes, is contemplated. In some instances, the isolated MSC exosomes may be administered continuously. Repeated or continuous administration may occur over a period of several hours (e.g., 1-2, 1-3, 1-6, 1-12, 1-18, or 1-24 hours), several days (e.g., 1-2, 1-3, 1-4, 1-5, 1-6 days, or 1-7 days) or several weeks (e.g., 1-2 weeks, 1-3 weeks, or 1-4 weeks) depending on the severity of the condition being treated. If administration is repeated but not continuous, the time in between administrations may be hours (e.g., 4 hours, 6 hours, or 12 hours), days (e.g., 1 day, 2 days, 3 days, 4 days, 5 days, or 6 days), or weeks (e.g., 1 week, 2 weeks, 3 weeks, or 4 weeks). The time between administrations may be the same or they may differ. As an example, if the symptoms of the disease appear to be worsening the α-type exosomes may be administered more frequently, and then once the symptoms are stabilized or diminishing the a-type exosomes may be administered less frequently.

In some embodiments, the isolated MSC exosomes are administered at least once within 24 hours of birth and then at least once more within 1 week of birth. In some embodiments, the isolated MSC exosomes are administered at least once within 1 hour of birth and then at least once more within 3-4 days of birth.

The isolated MSC exosomes may be administered by any route that effects delivery to the lungs or other tissues. Systemic administration routes such as intravenous bolus injection or continuous infusion are suitable. More direct routes such as intranasal administration, intratracheal administration (e.g., via intubation), and inhalation (e.g., via an aerosol through the mouth or nose) are also contemplated by the disclosure and in some instances may be more appropriate particularly where rapid action is necessary. As used herein, an aerosol is a suspension of liquid dispersed as small particles in a gas, and it includes a fine mist or a spray containing such particles. As used herein, aerosolization is the process of producing of an aerosol by transforming a liquid suspension into small particles or droplets. This may be done using an aerosol delivery system such as a pressurized pack or a nebulizer. Nebulizers include air-jet (i.e., pneumatic), ultrasonic, and vibrating-mesh nebulizers, for example with the use of a suitable propellant such as but not limited to dichlorodifluoromethane, trichlorofluoromethane, dichlorotetrafluoroethane, carbon dioxide or other suitable gas. In addition to nebulizers, other devices for pulmonary delivery include but are not limited to metered dose inhalers (MDIs) and dry powder inhalers (DPIs). Capsules and cartridges of for example gelatin for use in an inhaler or insufflator may be formulated containing lyophilized exosomes and a suitable powder base such as lactose or starch.

The isolated MSC exosomes, when it is desirable to deliver them systemically, may be formulated for parenteral administration by injection, including for example by bolus injection or continuous infusion. Formulations for injection may be presented in unit dosage form, e.g., in ampoules or in multi-dose containers, with or without an added preservative.
The compositions may take such forms as water-soluble suspensions, solutions or emulsions in oily or aqueous vehicles, and may contain formulatory agents such as suspending, stabilizing and/or dispersing agents. Suitable lipophilic solvents or vehicles include fatty oils such as sesame oil, or synthetic fatty acid esters, such as ethyl oleate or triglycerides. Aqueous injection suspensions may contain substances which increase the viscosity of the suspension, such as sodium carboxymethyl cellulose, sorbitol, or dextran. Optionally, the suspension may also contain suitable stabilizers or agents which increase solubility. Alternatively, the exosomes may be in lyophilized or other powder or solid form for constitution with a suitable vehicle, e.g., sterile pyrogen-free water, before use.

It is to be understood that other agents to be administered to subjects being treated according to the disclosure may be administered by any suitable route including oral administration, intranasal administration, intratracheal administration, inhalation, intravenous administration, etc. Those of ordinary skill in the art will know the customary routes of administration for such secondary agents.

In some embodiments, the isolated MSC exosomes, or the pharmaceutical composition comprising the MSC exosomes, when administered to a subject, improves lung function. Herein, lung function is considered to be "improved" when the functionality of the lung, e.g., as measured by any methods known to the skilled artisan, is improved by at least 20% in subjects that have been administered the isolated MSC exosomes, compared to in subjects that have not been administered the isolated MSC exosomes. For example, lung function may be considered improved when the functionality of the lung, e.g., as measured by any methods known to the skilled artisan, is improved by at least 20%, at least 30%, at least 40%, at least 50%, at least 70%, at least 80%, at least 90%, at least 100%, at least 2-fold, at least 5-fold, at least 10-fold, at least 50-fold, at least 100-fold or more, in subjects that have been administered the isolated MSC exosomes, compared to in subjects that have not been administered the isolated MSC exosomes. In some embodiments, lung function is considered to be improved when the functionality of the lung, e.g., as measured by any methods known to the skilled artisan, is improved by 20%, 30%, 40%, 50%, 70%, 80%, 90%, 100%, 2-fold, 5-fold, 10-fold, 50-fold, 100-fold or more, in subjects that have been administered the isolated MSC exosomes, compared to in subjects that have not been administered the isolated MSC exosomes.

Methods of evaluating lung function are known to those skilled in the art. Non-limiting examples of such methods include spirometry (measures the rate of air flow and estimates lung size), lung volume tests (measures how much air the lungs can hold), lung diffusion capacity (assesses how well oxygen gets into the blood from the air that is inhaled), pulse oximetry (estimates oxygen levels in blood), arterial blood gas tests (directly measures the levels of gases, such as oxygen and carbon dioxide, in blood), and fractional exhaled nitric oxide tests (measures how much nitric oxide is in the air that is exhaled).

In some embodiments, the isolated MSC exosomes, or the pharmaceutical composition comprising the MSC exosomes, when administered to a subject, restores lung architecture. Herein, lung architecture is considered to be "restored" when the architecture of the lung, e.g., as indicated by the percentage of alveoli with normal (e.g., as in a healthy subject) structure morphology, is increased by at least 20% in subjects that have been administered the isolated MSC exosomes, compared to in subjects that have not been administered the isolated MSC exosomes. For example, lung architecture may be considered "restored" when the architecture of the lung, e.g., as indicated by the percentage of alveoli with normal (e.g., as in a healthy subject) structure morphology, is improved by at least 20%, at least 30%, at least 40%, at least 50%, at least 70%, at least 80%, at least 90%, at least 100%, at least 2-fold, at least 5-fold, at least 10-fold, at least 50-fold, at least 100-fold or more, in subjects that have been administered the isolated MSC exosomes, compared to in subjects that have not been administered the isolated MSC exosomes. In some embodiments, lung architecture may be considered "restored" when the architecture of the lung, e.g., as indicated by the percentage of alveoli with normal (e.g., as in a healthy subject) structure morphology, is improved by 20%, 30%, 40%, 50%, 70%, 80%, 90%, 100%, 2-fold, 5-fold, 10-fold, 50-fold, 100-fold or more, in subjects that have been administered the isolated MSC exosomes, compared to in subjects that have not been administered the isolated MSC exosomes. The skilled artisan is familiar with methods of assessing lung architecture.

In some embodiments, the isolated MSC exosomes, or the pharmaceutical composition comprising the MSC exosomes, when administered to a subject, reduces pulmonary fibrosis. "Pulmonary fibrosis" refers to a condition where lung tissue becomes damaged and scarred, causing thickening and stiffing of the lung tissue and reduced lung function. Pulmonary fibrosis can have a variety of cause. Pulmonary fibrosis is typically seen in subjects with BPD. Herein, pulmonary fibrosis is considered "reduced" when the degree of pulmonary fibrosis (e.g., as indicated by collagen deposition on lung tissues) is reduced by at least 20%, in subjects that have been administered the isolated MSC exosomes, compared to in subjects that have not been administered the isolated MSC exosomes. For example, pulmonary fibrosis may be considered reduced when the degree of pulmonary fibrosis (e.g., as indicated by collagen deposition on lung tissues) is reduced by at least 20%, at least 30%, at least 40%, at least 50%, at least 60%, at least 70%, at least 80%, at least 90%, at least 95%, at least 99%, or 100%, in subjects that have been administered the isolated MSC exosomes, compared to in subjects that have not been administered the isolated MSC exosomes. In some embodiments, pulmonary fibrosis is considered reduced when the degree of pulmonary fibrosis (e.g., as indicated by collagen deposition on lung tissues) is reduced by 20%, 30%, 40%, 50%, 60%, 70%, 80%, 90%, 95%, 99%, or 100%, in subjects that have been administered the isolated MSC exosomes, compared to in subjects that have not been administered the isolated MSC exosomes.

In some embodiments, the isolated MSC exosomes, or the pharmaceutical composition comprising the MSC exosomes, when administered to a subject, reduces inflammation in the lung. One skilled in the art is familiar with methods of assessing the degree of inflammation in the lung, e.g., by measuring the levels of biomarkers of inflammation in the lung or in the blood. In some embodiments, inflammations in the lung is reduced by at least 20%, in subjects that have been administered the isolated MSC exosomes, compared to in subjects that have not been administered the isolated MSC exosomes. For example, inflammation may be reduced by at least 20%, at least 30%, at least 40%, at least 50%, at least 60%, at least 70%, at least 80%, at least 90%, at least 95%, at least 99%, or 100%, in subjects that have been administered the isolated MSC exosomes, compared to in subjects that have not been administered the isolated MSC exosomes. In some embodiments, inflammation is reduced by 20%, 30%, 40%, 50%, 60%, 70%, 80%, 90%, 95%, 99%, or 100%, in subjects that have been administered the isolated MSC exosomes, compared to in subjects that have not been administered the isolated MSC exosomes. Without wishing to be bound by scientific theory, the isolated MSC exosomes described herein are shown to have immunomodulatory activities, possibly via regulating the expression of genes involved in immune response and inflammation, and/or regulating macrophage polarization. "Immunomodulatory activity" refers to the activity of the isolated MSC exosomes described in modulating (e.g., enhancing or reducing) the immune response and/or inflammatory response. As described herein, the isolated MSC exosome downregulate the expression of various pro-inflammatory genes (e.g., in Figure 9E).

In some embodiments, isolated MSC exosomes, or the pharmaceutical composition comprising the MSC exosomes, when administered to a subject, reduces peripheral pulmonary arterial remodeling. "Peripheral pulmonary arterial remodeling" is an important pathological feature of pulmonary arterial hypertension (PAH), which leads to increased pulmonary vascular resistance and reduced compliance, with marked proliferation of pulmonary artery smooth muscle cells (SMC) and/or endothelial cells (EC) resulting in the obstruction of blood flow in the resistance pulmonary arteries. Certain forms of pulmonary arterial remodeling is reversible. "Reducing" pulmonary arterial remodeling may be achieved by either preventing new remodeling, or reverse old remodeling. One skilled in the art is familiar with methods of assessing the degree of pulmonary arterial remodeling. In some embodiments, pulmonary arterial remodeling is reduced by at least 20%, in subjects that have been administered the isolated MSC exosomes, compared to in subjects that have not been administered the isolated MSC exosomes. For example, pulmonary arterial remodeling may be reduced by at least 20%, at least 30%, at least 40%, at least 50%, at least 60%, at least 70%, at least 80%, at least 90%, at least 95%, at least 99%, or 100%, in subjects that have been administered the isolated MSC exosomes, compared to in subjects that have not been administered the isolated MSC exosomes. In some embodiments, pulmonary arterial remodeling is reduced by 20%, 30%, 40%, 50%, 60%, 70%, 80%, 90%, 95%, 99%, or 100%, in subjects that have been administered the isolated MSC exosomes, compared to in subjects that have not been administered the isolated MSC exosomes.

Other aspects of the present disclosure provide methods of isolating the MSC exosomes described herein. The isolated MSC exosome is substantially free of other substances, e.g., protein contaminants, and retains the therapeutic efficacy of MSC exosomes. As described herein (e.g., in Figures 1A and 1B), the culturing media of MSCs (e.g., MSCs from Wharton's jelly or bone marrow) are concentrated (e.g., via tangential flow filtration) and subjected to fractionation through an iodixanol (IDX) cushion of 10%-55% gradient. The fractionation is achieved by floating the concentrated media on top of the IDX cushion and ultracentrifugation of the mixture. The fractions are collected as indicated in Figure 1B, and fraction 9 contains the isolated MSC exosomes described herein. The MSC exosomes in fraction 9 are lower in protein contents and high in MSC exosome contents. In some embodiments, the MSC exosomes in fraction 9 are substantially free of non-exosomal protein contaminants.

The isolated MSC exosomes (fraction 9) may be used immediately (e.g., in the methods described herein) or alternatively, the isolated MSC exosomes may be stored short- and/or long-term, e.g., in a cryopreserved state prior to use. Proteinase inhibitors are typically included in freezing media as they provide exosome integrity during long-term storage. Freezing at -20°C is not preferable since it is associated with increased loss of exosome activity. Quick freezing at -80°C is more preferred as it preserves activity. (See for example Kidney International (2006) 69, 1471-1476.) Additives to the freezing media may be used in order to enhance preservation of exosome biological activity. Such additives will be similar to the ones used for cryopreservation of intact cells and may include, but are not limited to DMSO, glycerol and polyethylene glycol.

Some of the embodiments, advantages, features, and uses of the technology disclosed herein will be more fully understood from the Examples below. The Examples are intended to illustrate some of the benefits of the present disclosure and to describe particular embodiments, but are not intended to exemplify the full scope of the disclosure and, accordingly, do not limit the scope of the disclosure.

### EXAMPLES

The presently claimed invention is as defined in the claims. Any of the following Examples that do not fall within the scope of the claims do not form part of the presently claimed invention and are provided for comparative purposes only.

### Example 1: Stem Cell Exosomes Ameliorate Bronchopulmonary Dysplasia and Restore Lung Function through Macrophage Immunomodulation.

Bronchopulmonary dysplasia (BPD) is a chronic lung disease that occurs almost exclusively in preterm infants who have required mechanical ventilation and oxygen therapy. It is characterized by restricted lung growth (tissue simplification), subdued alveolar and blood vessel development and impaired pulmonary function. Several BPD outcomes are associated with long-term pulmonary complications, such as abnormal pulmonary function test results, altered airway hyper-responsiveness and, in moderate to severe cases, secondary pulmonary hypertension (PH) (1-5). With no effective single therapy for preventing or treating developmental lung injuries, the need for new tools to treat and reduce burden of such lifelong complications associated with extreme preterm birth is urgent.

In addition to restricted pulmonary development, several investigators have noted a loss of stem/progenitor cell populations that may underlie the observed tissue simplification (6-8). Such findings have provided a platform for the application of stem cell-based therapies to ameliorate lung injury associated with preterm birth. Administration of different stem/progenitor cell types, such as mesenchymal stem cells (MSCs), (9-11) endothelial colony forming cells (ECFCs) (12) and human amnion epithelial cells (hAECs), (13) have shown promise in preclinical models for the prevention and/or treatment of BPD and other major sequelae of prematurity(14, 15).

Despite substantial physiologic improvements in the recipient lung following MSC therapy, several preclinical studies noted the absence of significant engraftment of donor cells in the lung, suggesting the therapeutic mechanism of action may be paracrine or indirect (16, 17). Indeed, previous work (9, 18,19, and 20) has demonstrated that the cell-free conditioned media (CM) of MSCs afforded superior protection over MSCs themselves in preserving the lung architecture and preventing alveolar loss in preclinical BPD models. Mesenchymal stem cell (MSC) therapy has shown promise in preclinical models of BPD, and recent studies established that one of the main therapeutic vectors of MSCs is comprised in their secretome and represented by exosomes (extracellular vesicles; EVs) and, in particular, the smaller (less than 150nm diameter) subpopulation generated through the endocytic pathway, termed exosomes (19).

Noting the therapeutic capacity of MSCs in the lung is comprised in their secretome, previous research has shown that the therapeutic vector is represented by the EVs they release (21-23). Beneficial properties of MSC-EV treatment have also been reported for a number of disease models, including myocardial infarction (24, 25), kidney injury, (26, 27), modulating of immune responses (28, 29) and neurological protection (30, 31); for a recent review see (32). Yet, more often than not, crude EV isolation techniques coupled with poor analytical characterization obfuscates the therapeutic impact of MSC-EVs, impairing bioavailability and co-contaminating preparations with non-EV material. A detailed characterization of purified MSC-EVs from both human umbilical cord Wharton's jelly (WJMSCs) and bone marrow MSCs (BMSCs) are described herein. Further, the efficacy of MSC-EV treatments in experimental models of BPD is investigated. The results outlined herein show that a bolus dose of purified MSC- EVs significantly improves lung morphology and pulmonary development, decreases lung fibrosis, and ameliorates pulmonary vascular remodeling in a hyperoxia-induced model of BPD. It is further shown that MSC-EV treatment improves pulmonary function test results and alleviates associated pulmonary hypertension. Furthermore, this study demonstrates MSC-EV treatment blunts hyperoxia-induced inflammation, an effect that, in part, is via modulation of lung macrophage polarization.

Herein, exosomes (EVs 30-150 nm in diameter, expressing markers CD9, CD63 & Flotillin-1 and floating at a density of ~1.18g/ml) were isolated from cell culture supernatants (CM) after 36 hours incubation in serum-free-media (SFM). Following differential centrifugation to clarify cell debris and related apoptotic detritus, CM were concentrated by filtration and exosomes isolated by flotation on an OptiPrep^{™} (iodixanol; IDX) cushion and further characterized. See online data supplement for details.

ANOVA followed by Bonferroni's multiple comparison test (GraphPad, v 6.0, CA, US) was used for statistical analysis. Pearson correlation coefficients were used to explore the strength of the relationship between immunohistochemistry vascular remodeling parameters and physiological indices of PH. Flow cytometry data analyses utilized FlowJo software v10.2 (TreeStar, OR, US). Inflammatory marker mRNA levels were assessed by RT-qPCR and expressed relative to cognate NRMX control group average level; significance was considered at p < 0.05.

For in vivo studies, sample size calculations were based on previous work (14), suggesting that detection of a 15% improvement in lung architecture (assessed by mean linear intercept; MLI), with >90% power at the 5% α-level, required a minimum of 5 animals per group.

Investigators were blinded to experimental groups for histological analysis and physiological measurements.

### Purification, isolation, and characterization of MSC-EVs

Human MSCs secrete a diverse vesicle population. Many EV isolation methods often contaminate EV preparations with protein, reduce EV bioavailability and promote aggregates (33). Thus, the aim was to isolate and purify EVs from concentrated MSC-CM by iodixanol density gradient in an attempt to overcome the aforementioned limitations of conventional EV isolation techniques **(****Figures 1A** and **1B****).** Flotation of CM from WJMSCs, BMSCs or human dermal fibroblasts (HDFs) cultures on an IDX cushion allowed for the extraction of EVs in fraction 9 of the gradient. Comparing the vesicles that were assessed between fraction 6-10 by transmission electron microscopy (TEM) and nanoparticle tracking analysis (NTA), fraction 9 boasted a superior protein: particle ratio that corresponded to a density of -1.18 g/ml **(Figure 1C).**

TEM and NTA analysis of fraction 9 revealed a heterogeneous EV population for WJMSC-EV, BMSC-EV, and HDF-EV samples, that occupied a typical diameter of 50-150 nm, had minimal protein contaminants, and exhibited the distinct biconcave morphological features of EVs **(****Figures 1D** and **1E****).** Immunoblots of IDX cushion gradients revealed fraction 9 for all cell types was positive for CD9, CD63, and flotillin-1 (Flot-1, **Figure 1F**).

### Short term assessment (PN14): A single dose of MSC-EV-treatment restores lung architecture

Newborn mice were exposed to HYRX (75% O₂) for 7 days (postnatal day (PN) 1-7) before being returned to room air for a further 7 days. HYRX-exposed mice were compared to mice that remained at NRMX (room air). Treatment groups received a single intravenous (IV) dose of WJMSC-EVs, BMSC-EVs, or HDF-EVs after HYRX exposure had commenced at PN4 (schematic shown in **Figure 2A****).** Compared to NRMX-control mice, HYRX-exposed mice demonstrated a histological pattern reminiscent of human BPD, characterized by severe impairment of the alveolar growth, large airspaces and incomplete alveolar septation **(****Figure 2B****).** At PN14 the mean linear intercept (MLI) value was elevated in HYRX-control mice when compared to NRMX group (38.7 ± 1 vs. 25.1 ± 1, p < 0.0001, respectively). Animals treated with MSC-EVs presented with dramatically improved alveolarization and almost completely restored lung architecture compared to the HYRX-control group. This improvement was independent of MSC origin and was observed in treatments with either WJMSC-EVs (29.8 ± 1, p < 0.001) and BMSC-EVs (31.6 ± 2, p < 0.001). HDF-EVs served as a biologic and vehicle control and demonstrated no significant protective effect at PN14 (34.24 ± 2 µm, p > 0.05), thus were not utilized for experiments assessing long-term effects. No difference was found comparing NRMX-control animals and animals treated with WJMSC-exos under normoxia (figure E1, p > 0.05).

### Long term assessment (PN42): A single dose of MSC-EV-treatment improves pulmonary development and ameliorates septal fibrosis

To assess long-term endpoints, lung architecture and collagen deposition was assessed at PN42 **(****Figure 3****).** HYRX-control mice demonstrated persistent destruction of the alveolar architecture, large airspaces, and significant alveolar simplification compared to NRMX control group (MLI=26.7 ± 0.7 vs. 18.8 ± 0.5 µm, p < 0.0001). Again, animals treated with either WJMSC- EVs or BMSC-EVs presented a dramatically improved alveolarization and robust restoration of lung architecture when compared to HYRX-control mice (WJMSC-EV: MLI=21.6 ± 0.9 µm, p < 0.01; BMSC-EV: MLI=22.7 ± 0.4 µm, p < 0.01).

Collagen deposition (Masson's trichrome stain) was assessed at PN42 to measure the degree of lung fibrosis. Slight collagen deposition was recorded in all groups in this model of BPD (<1% of total septal area), in comparison to more severe models of BPD where animals were exposed to HYRX for 14 days (18). However, HYRX-control mice had a subtle but significantly higher amount of collagen deposition compared to NRMX-control mice (0.22 ± 0.06 vs. 0.08 ± 0.01 µm; p < 0.01, respectively). HYRX-exposed mice treated with either WJMSC-EVs or BMSC- EVs had significantly decreased collagen deposition (0.1 ± 0.02 and 0.13 ± 0.01 µm; p < 0.01, p < 0.05, respectively, **Figure 3****).**

### MSC-EV treatment rescues hyperoxia-induced loss of peripheral pulmonary blood vessels and peripheral pulmonary arterial remodeling

To explore the potential impact of MSC-EVs on HYRX-induced peripheral pulmonary vascular remodeling, lung sections of mice harvested at PN42 were stained with α-smooth muscle actin (α-SMA, **Figure 4A****).** HYRX-exposed animals demonstrated a greater peripheral pulmonary vascular muscularization (36.55 ± 2.7 media thickness index (MTI)) compared to NRMX-control animals (20.23 ± 2.2 MTI, p < 0.01). With little evidence to suggest differences between WJMSC-EVs and BMSC-EVs in regards to long-term lung architecture improvements and reducing fibrosis, the MSC-EV groups were combined. MSC-EV treatment ameliorated the HYRX-induced vascular muscularization (26.8 ± 1.3 MTI, p < 0.01, **Figure 4B****).** WJMSC-exo and BMSC-exo treatment ameliorated the HYRX-induced vascular muscularization (26.3 ± 1.1 MTI, p < 0.05 and 27.22 ± 2.3 MTI, p < 0.05 respectively, **Figure 13A****).**

To quantify the degree of HYRX-induced PH, direct right ventricular systolic pressure (RVSP) measurements, the right ventricle to body weight ratio (RV:BW) and Fulton's index were assessed. Compared with NRMX-controls, RVSP was found to be modestly elevated in HYRX- control animals (22.38 ± 0.68 vs. 30.8 ± 0.85 mmHg, p < 0.001, respectively). MSC-EV treatment ameliorated this modest elevation in RVSP (26.85 ± 0.83 mmHg, p < 0.05, **Figure 4C** **and** **Figure 13C****).** The same trend was recapitulated on assessment of the RV:BW ratio and Fulton's index, where compared to their NRMX counterparts, HYRX-control mice presented with an elevated RV:BW ratio (7.7 ± 0.01 × 10⁻⁴ vs. 8.5 ± 0.02 × 10⁻⁴, p < 0.05, respectively) and Fulton's index (0.22 ± 0.007 vs. 0.26 ± 0.015, p < 0.05, respectively, **Figure 13D****).** MSC-EV treatment ameliorated the modest increase in RV:BW ratio (7.7 ± 0.01× 10⁻⁴, p < 0.05, **Figure 4D****)** and Fulton's index (0.23 ± 0.027, p < 0.05), although the trend in Fulton's index did not reach significance (not shown). Across all experimental groups, the degree of vascular remodeling (α-SMA staining) correlated significantly with RV:BW (p = 0.002, r = 0.807), Fulton's index (p = 0.001, r = 0.69) and RVSP (p = 0.002, r = 0.806).

To determine the effect of HYRX-exposure on pulmonary vessel number, von Willebrand factor (vWF)-staining in lung sections of mice at PN42 were assessed. Compared to NRMX-control mice, HYRX exposure led to significant loss of small (peripheral) vessels <50 µm diameter (4.4 ± 0.3 vs. 2.9 ± 0.2 blood vessels/field (VPF) respectively, p < 0.01), whereas WJMSC-exo or BMSC-exo treatment restored these vessels (3.9 ± 0.4 and 4.4 ± 0.6 VPF, p = 0.06, and p < 0.01, respectively **(****Figure 4H** and **Figure 13B****).** There was no significant difference in number of vessels >150 µm diameter between the groups (not shown).

### MSC-EV treatment improves lung function following hyperoxia-induced lung injury

To determine the functional impact of altered tissue architecture and fibrosis, pulmonary function testing was next performed in the experimental groups (specifically NRMX-controls, HYRX-controls, and HYRX-exposed mice treated with WJMSC-EVs) at PN42. All animals were ventilated at a positive end expiratory pressure (PEEP) of 3 cm H₂O. In accordance with the lung histology (simplified alveolarization and emphysema-like features of lung disease), HYRX-exposed mice displayed an altered pressure-volume (PV)-loop when compared to the NRMX-control group. PV-loops in the HYRX-control group were shifted upwards and to the left, indicative of emphysema-like features of lung disease and air trapping (34). HYRX-control mice had an elevated total lung capacity (TLC, 1.36 ± 0.04 ml), compared to the NRMX-control group (0.77 ± 0.04 ml, p < 0.0001), which was ameliorated by WJMSC-EV treatment (1.18 ± 0.02 ml, p < 0.01, **Figure 4E****).** MSC-EV treatment improved the HYRX-induced emphysematous change, significantly shifting the PV-loop downwards and to the right **(****Figure 4F****).** Baseline measurements revealed that the airway resistance was not different between the three groups; however HYRX-control group had an elevated baseline compliance measurement that was not affected by MSC-EV treatment.

### MSC-EVs modulate the lung transcriptome and blunt hyperoxia-induced inflammation

Whole-organ RNA sequencing was used to generate an unbiased overview of the impact of MSC-EVs on the lung transcriptome **(****Figures 9A-9F****).** Total lung RNA from WJMSC-EV treatments were compared to NRMX and HYRX control groups. At PN7, HYRX-exposed animals had 2561 significantly enriched and 2344 suppressed mRNAs compared to their NRMX counterparts. WJMSC-EV treatment significantly suppressed a subset of the HYRX-induced genes (117 mRNAs), and significantly up-regulated 83 genes that were suppressed by HYRX exposure, shifting the overall gene expression profile towards their NRMX counterparts **(****Figure 5A****).** Interestingly, 41 genes were specifically induced by WJMSC-EV treatment and not by HYRX. Gene ontology (GO) analysis indicated these genes are likely involved in extracellular matrix and structural organization, cardiovascular development/morphogenesis, and SMAD protein import into the nucleus **(****Figure 9C****).** The tabulation of Plasma membered- associated proteins in the F9 fractions are given in Tables 1 and 2. The GO classifications are as follows: Integral Component of Membrane (GO: 0016021) and Anchored component of membrane (GO:0031225).

**Table 1: MEX-Specific Membrane Proteins: Present in purified MSC exosomes and absent in Dermal Fibroblast exosomes.**

| | **Normalized Peptide Number** | | |
|---|---|---|---|
| **Gene Symbol** | **WJ_MEX** | **F_MEX** | **Entrez Gene Name** |
| FLT1 | 13 | 0 | fms related tyrosine kinase 1 |
| PLAUR | 12 | 0 | plasminogen activator, urokinase receptor |
| MME | 12 | 0 | membrane metallo-endopeptidase |
| CDH2 | 12 | 0 | cadherin 2 |
| SLC16A3 | 10 | 0 | solute carrier family 16 (monocarboxylate transporter), member 3 |
| CDH13 | 8 | 0 | cadherin 13 |
| ITGB5 | 8 | 0 | integrin subunit beta 5 |
| ITGA11 | 7 | 0 | integrin subunit alpha 11 |
| APP | 6 | 0 | amyloid beta precursor protein |
| GPC6 | 6 | 0 | glypican 6 |
| IGSF8 | 6 | 0 | immunoglobulin superfamily member 8 |
| NRP2 | 5 | 0 | neuropilin 2 |
| TSPAN9 | 5 | 0 | tetraspanin 9 |
| DAG1 | 4 | 0 | dystroglycan 1 |
| SLC38A2 | 4 | 0 | solute carrier family 38 member 2 |
| SLC12A8 | 4 | 0 | solute carrier family 12, member 8 |
| GJA1 | 4 | 0 | gap junction protein alpha 1 |
| ITGA1 | 3 | 0 | integrin subunit alpha 1 |
| PLXNB2 | 3 | 0 | plexin B2 |
| SLC16A1 | 3 | 0 | solute carrier family 16 (monocarboxylate transporter), member 1 |
| PROCR | 3 | 0 | protein C receptor |

**Table 2: Membrane proteins enriched >4-fold in purified MSC exosomes compared to Dermal Fibroblast exosome.**

| | **Normalized Peptide Number** | | |
|---|---|---|---|
| **Gene Symbol** | **WJ_MEX** | **F_MEX** | **Entrez Gene Name** |
| PTK7 | 30 | 2 | protein tyrosine kinase 7 (inactive) |
| CD 109 | 18 | 4 | CD109 molecule |
| SLC1A5 | 16 | 3 | solute carrier family 1 (neutral amino acid transporter), member 5 |
| ITGA3 | 14 | 3 | integrin subunit alpha 3 |
| ITGA2 | 13 | 1 | integrin subunit alpha 2 |
| BSG | 13 | 3 | basigin (Ok blood group) |
| DPP4 | 8 | 2 | dipeptidyl-peptidase 4 |
| ATP1A1 | 8 | 2 | ATPase, Na+/K+ transporting, alpha 1 polypeptide |
| FAP | 7 | 1 | fibroblast activation protein alpha |
| VASN | 6 | 1 | vasorin |
| IGF2R | 6 | 1 | insulin like growth factor 2 receptor |
| CD82 | 5 | 1 | CD82 molecule |

**Table 3: MEX□Specific Membrane Proteins: Present in purified MSC exosomes and absent in Dermal Fibroblast exosomes**

| | **Normalized Peptide Number** | | |
|---|---|---|---|
| **Gene Symbol** | **WJ_MEX** | **F_MEX** | **Entrez Gene Name** |
| FLT1 | 13 | 0 | fms related tyrosine kinase 1 |
| PLAUR | 12 | 0 | plasminogen activator, urokinase receptor |
| MME | 12 | 0 | membrane metallo-endopeptidase |
| CDH2 | 12 | 0 | cadherin 2 |
| SLC16A3 | 10 | 0 | solute carrier family 16 (monocarboxylate transporter), member 3 |
| CDH13 | 8 | 0 | cadherin 13 |
| ITGB5 | 8 | 0 | integrin subunit beta 5 |
| ITGA11 | 7 | 0 | integrin subunit alpha 11 |
| APP | 6 | 0 | amyloid beta precursor protein |
| GPC6 | 6 | 0 | glypican 6 |
| IGSF8 | 6 | 0 | immunoglobulin superfamily member 8 |
| NRP2 | 5 | 0 | neuropilin 2 |
| TSPAN9 | 5 | 0 | tetraspanin 9 |
| DAG1 | 4 | 0 | dystroglycan 1 |
| SLC38A2 | 4 | 0 | solute carrier family 38 member 2 |
| SLC12A8 | 4 | 0 | solute carrier family 12, member 8 |
| GJA1 | 4 | 0 | gap junction protein alpha 1 |
| ITGA1 | 3 | 0 | integrin subunit alpha 1 |
| PLXNB2 | 3 | 0 | plexin B2 |
| SLC16A1 | 3 | 0 | solute carrier family 16 (monocarboxylate transporter), member 1 |
| PROCR | 3 | 0 | protein C receptor |

Using GO analysis it was found that HYRX-exposure upregulated genes involved in the adaptive immune response, inflammatory response and leukocyte mediated immunity **(****Figure 9D****).** WJMSC-EV treatment blunted the induction of proinflammatory HYRX-responsive genes, suppressing genes involved in inflammation, adaptive immune responses, IFN-γ mediated-signaling, granulocyte production, and leukocyte chemotaxis and cytokine production **(****Figure 9E****).** In contrast to PN7, fewer changes in the lung mRNA profile were detected at PN14 **(****Figure 9B****).** HYRX control mice had 356 significantly enriched and 282 suppressed mRNAs when compared to their NRMX counterparts. WJMSC-EV treated mice continued to significantly modulate levels of a subset of HYRX-dysregulated genes **(****Figure 9F**).

Validation of selected whole-lung RNA-seq data was assessed by RT-qPCR. In accordance, at PN7 proinflammatory markers and indices of the classically-activated M1 macrophage, such as Ccl2, Ccl7 and Il6 were elevated in the HYRX-control group compared to NRMX-control mice, and were significantly suppressed by MSC-EV treatment **(****Figure 5B****).** Furthermore, genes associated with the anti-inflammatory M2-like polarization, such as arginase-1 (Arg1), Cd206 and Ccl17, were also dysregulated in the HYRX-control group, and were effectively modulated by MSC-EV treatment **(****Figure 5B****).** Graphs depicting the effect of either WJMSC-exo or BMSC-exo preparations can be found in Figure 14.

### MSC-EVs suppress inflammation by immunomodulation of macrophage phenotype in vitro and in vivo

Macrophage polarization plays an important role in regulating the immune response and inflammation in the developing lung (35). The whole-lung RNA analysis demonstrates that MSC-EVs modulate the expression of HYRX-induced inflammatory genes, suggesting that EVs might provide a therapeutic benefit for BPD by regulating immune cell function. To test whether EVs can directly modulate immune function, next the ability of EVs to regulate macrophage phenotypes *in vitro* was assessed. Using fluorescence microscopy it was observed that WJMSC-EVs were readily taken up by alveolar macrophages **(****Figure 10A****).** It was then determined whether WJMSC-EVs could regulate key macrophage genes by RT-qPCR. In a dose-dependent manner, the addition of WJMSC-EVs to classically-activated (M1) macrophages significantly reduced the mRNA levels of established proinflammatory M1 markers such as TNFα, Il6, and Ccl5, p < 0.05 **(****Figures 6A** and **10B****).** The addition of WJMSC-EVs to M2 polarized macrophages significantly suppressed Retnla and Cd206 induction (p < 0.001 and p < 0.01, respectively, **Figure 6B****),** and, most importantly, greatly enhanced macrophage Arg1 expression levels. This is a direct reflection of the MSC-EVs anti-inflammatory action, since depletion of arginine, the iNOS substrate, will effectively diminish M1 functionally of the macrophages, buttressing thus the M2-like state. HDF-EVs were used as a vehicle and biologic control and demonstrated minimal effect.

In the HYRX-induced BPD model described herein, flow cytometry was used to assess lung macrophage (Cd45^{+ve}, Cd11b^{-ve}, Cd11c^{+ve}, Cd64^{+ve}) phenotype *in vivo* at PN7 and PN14 **(****Figure 6C, 6D****,** and **11****).** Cd40 and Cd86 were used as proinflammatory M1 markers whilst Cd206 was used to identify the alternatively activated (M2-like) macrophages. Akin to in *vitro* experiments and *in vivo* lung mRNA levels, WJMSC-EVs significantly suppressed the HYRX-induced increase in Cd206 levels at PN7 (p < 0.05), a trend which continued to PN14 (p < 0.01). At PN7 and PN14, Cd40 levels were elevated in HYRX-exposed mice compared to their NRMX counterparts. This increase was suppressed by WJMSC-EV treatment at PN14, although a similar trend was observed at PN7, this was non-significant. No difference in Cd86 levels across all groups at both PN7 and PN14 was found.

### Discussion

Several animal models have been developed, and continue to be refined, aiming to recapitulate pathological pulmonary hallmarks noted in lungs of human neonates with BPD. Although every preclinical model of BPD is subject to its own advantages and limitations, arguably BPD is most commonly modeled in mice. Reasons for this are manifold, and include relatively short gestation times allowing expedient studies on lung development. Importantly, the saccular stage of murine lung development occurs between E17.5 and PN5. Therefore, term mouse lungs present a development stage resembling that of a human preterm neonate between 24 and 28 weeks gestation. This represents an excellent model for developmental lung injury, recently reviewed in (27). The model used in this study presents significant alveolar simplification and subtle elevation in indices of secondary PH, closely resembling the human phenotype of "new" BPD.

The study described herein shows that a bolus dose of purified human MSC-EVs effectively alleviates and rescues core features of HYRX-induced BPD, even after the injury has commenced. Specifically, the study described herein is the first to show that MSC-EV treatment radically improved lung morphology and pulmonary development, decreased lung fibrosis, rescued pulmonary vasculature loss, and ameliorated pulmonary vascular remodeling. These observations were extended to show that the cytoprotective actions of MSC-EVs results in desirable functional outcomes such as improved pulmonary function test results and amelioration of associated PH. Furthermore it was found that MSC-EVs blunt HYRX-induced proinflammatory signaling and immune responses that may, in part, be via modulation of lung macrophage phenotype.

Previous studies have shown that the administration of the MSC secretome partially prevented and/or reversed parenchymal fibrosis and peripheral pulmonary artery devascularization, ameliorated alveolarization, and improved lung function test results in experimental models of BPD (18, 20). However, the biological mediators responsible for the MSC secretome remained elusive. In accordance, this data supports and extends findings to show that the therapeutic vector within MSC-CM is harnessed by the EVs and that a single dose of MSC-EVs is able to ameliorate HYRX-induced alveolar simplification, improve vascular remodeling, pulmonary blood vessel loss, and blunt lung fibrosis. Previous studies have revealed that infants with BPD exhibit abnormalities in lung function that begin in infancy and often persist throughout childhood, and even into early adolescence. Consequently, adults who were former BPD infants may be at increased risk of developing chronic obstructive pulmonary disease (COPD) (36). Complementing the histological findings of almost completely restored lung architecture, vascular remodeling and hemodynamics, this study also shows that MSC-EV treatment had a significant long-term functional benefit to pulmonary mechanics, improving PV-loops and reducing TLC.

Although neutrophil activation is an important contributor to pulmonary inflammation (37), macrophages are critical mediators of the neonatal lung immune response, facilitating both the initiation and the resolution of inflammation (38, 39). MSC-EVs were shown to be immunomodulatory in models of immune disease and to regulate macrophage phenotypes (28, 29, 40). Macrophage phenotypes are diverse, representing the impact of multidimensional networks on development, activation and functional diversity (37, 38). The traditional M1/M2 binary paradigm is considered obsolete (39) and, indeed, recent elegant studies in preclinical models stress the effects of microenvironment on pulmonary macrophage plasticity and address the diversity of temporal and compartmental macrophage phenotypes in relation to lung homeostasis and disease (35, 36, 40).

Using MSC-macrophage co-cultures it was shown that the MSC secretome is a major paracrine mediator in the M1 to M2 shift. Notably, chronic M2 activation could catalyze detrimental conditions of fibrosis where amplified tissue remodeling could prevent optimal repair (38, 41). In accordance, Sicco and colleagues reported that the addition of MSC-EVs to macrophages elicits the switch from a proinflammatory M1 phenotype towards a M2-like state, and that MSC-EVs modulated tissue mRNA levels of M1 and M2 markers in a cardiotoxin- induced muscle injury model (42).

Observations from this study provide evidence that MSC-EVs modulate the M1/M2 phenotype fulcrum, suppressing the proinflammatory M1 state and modulating the anti-inflammatory M2-like polarization both *in vitro* and *in vivo.* Directly related to the MSC-EV anti-inflammatory and immunomodulatory capacity, MSC-EV treatment had pleiotropic effects that collectively modulate most, but not all of the HYRX-induced signaling that may underlie the developmental arrest in the neonatal lung. Previously, preclinical models of HYRX-induced neonatal lung injury (43) as well as clinical studies (44) have shown inflammatory pathways to be dysregulated in BPD. Specifically, a study looking at 1067 extremely low birth weight infants found that higher concentrations of IL-1β, -6, -8, -10, and IFN-γ were associated with BPD and infant death (45). Taken together, whole lung transcriptome analysis coupled with *in vitro* and *in vivo* assessment of macrophage polarization demonstrates MSC-EVs harness potent immunomodulatory capabilities, able to act at the peak of inflammatory state, during the hyperoxic insult. At PN14, after exposed animals had returned to room air for a week, there was evidence to suggest a degree of recovery and normalization of some, but not all HYRX-induced dysregulation of gene expression. Thus, early intervention and blunting the HYRX-induced inflammation process appears to be critical for preserving lung development. At PN14, when exposed animals had already been returned to room air for a week, most (albeit not all) of the hyperoxia-induced dysregulation of lung gene expression had returned to normal, suggesting that early intervention and blunting the early hyperoxia-induced inflammatory phase is critical for preserving proper lung development.

Robust physiological changes were observed following MSC-EV treatment. The MSC EV dose based was estimated on prior literature using MSC-EVs in an adult murine model of hypoxia-induced PH (21). Future studies will further investigate dose responses and different routes of administration.

In summary, as the incidence of chronic neonatal lung disease continues to rise, it is clear that EV-therapeutics may represent a major paradigm for diseases of the newborn infant. In this study EVs-derived from human WJMSCs, BMSCs, and HDF-EVs were isolated, identified, and comprehensively characterized. Additionally, a robust therapeutic effect that is unique to MSC-EVs versus vesicles derived from HDFs is demonstrated herein. Importantly, this represents the first time that purified MSC-EVs are demonstrated to be the major paracrine anti-inflammatory and therapeutic mediators of MSC action in the lung that may act, at least in part, through modulation of the lung macrophage phenotype, suppressing lung inflammation and immune responses to favor tissue development. Given the recognized pleiotropic effects of MSC-EVs (46), other prematurity-associated pathologies, including neurological impairment, could be the targets of exosome-based therapies.

### Methods

### Animal model and experimental design

Extended description of the hyperoxia-induced BPD model, experimental design and standard analytical methods are described in the supplemental methods. All animal experiments were approved by the Boston Children's Hospital Animal Care and Use Committee.

### Exosome isolation, purification and characterization

EVs were isolated directly from cell culture supernatants. Cell culture media was harvested after 36 hours incubation in serum-free-media (SFM) to avoid EV contamination from FBS. Cell culture media were subjected to differential centrifugation, 300 × *g* for 10 minutes (to remove any cells in suspension) followed by 3000 × *g* for 10 minutes and 13,000 × *g* for 30 minutes to remove any cell debris and large apoptotic bodies in suspension **(****Figure 1A****).** MSC-CM was concentrated 50-fold by tangential flow filtration (TFF) using a modified polyethersulfone (mPES) hollow fiber with 300 kDa MW cutoff (Spectrum Labs, Irving TX). EVs were further purified using OPTIPREP^{™} (iodixanol; IDX) cushion density flotation. An IDX gradient was carefully prepared by floating 3 ml of 10% w/v IDX solution containing NaCl (150 mM) and 25 mM Tris:HCl (pH 7.4) over 3 ml of 55% w/v IDX solution. Concentrated CM (6 ml) was floated on top of the IDX cushion and ultracentrifuged using a SW 40 Ti rotor for 3.5 hours at 100,000 × *g* (4 °C, **Figure 1B****).** Twelve fractions (1 ml each) were collected from the top of the gradient for immediate EV characterization or frozen (-1 °C/min) and kept at -80 °C. IDX fraction density was assessed by weight/volume ratio (g/ml).

### Statistics

Data between different groups was compared by ANOVA followed by Bonferroni's multiple comparison test using GraphPad Prism (v6.0; GraphPad, CA, US). Pearson correlation coefficients were used to explore the strength of the relationship between immunohistochemistry vascular remodeling parameters and physiological indices of PH. Flow cytometry data analyses were performed using FlowJo software v10.2 (TreeStar, OR, US). Values are expressed as mean ± SEM unless otherwise stated. Assessment of inflammatory marker mRNA levels by RT-qPCR are expressed as relative to their cognate NRMX control group and are presented as median ± range. Significance was considered at *p* < 0.05 unless stated otherwise. For *in vivo* studies, the sample size calculations were based on previous work (18), suggesting that detection of a 15% improvement in lung architecture (assessed by mean linear intercept; MLI), with >90% power at the 5% α-level, requires a minimum of 5 animals per group.

### Cell isolation and culture

Human umbilical cord Wharton's jelly mesenchymal stem cells (WJMSCs) were isolated using a modification of the explant method (47). Briefly, the umbilical cord was rinsed twice with Dulbecco's phosphate-buffered saline (dPBS, Invitrogen, MA, US), cut longitudinally, and arteries and vein were removed. The soft gel tissues were finely dissected into small pieces (~3-6 mm²) and individually placed on 193 mm² tissue culture dishes (24-well plate) with α-Modified Eagle Medium (αMEM, Invitrogen, MA, US) supplemented with 20% fetal bovine serum (FBS, Invitrogen, MA, US), 2 mM L-glutamine, and penicillin/streptomycin, and incubated for 12 days at 37°C in a humidified atmosphere of 5% CO₂. After periodic addition of supplemented αMEM, umbilical cord tissue was carefully removed. Plates were washed 3 times with media; the plastic adherent cell colonies were trypsinized and maintained in culture in αMEM, supplemented with 10% fetal bovine serum, 2 mM L-glutamine, and penicillin/streptomycin. At passage 4, WJMSCs were expanded to 10-stack Corning CELLSTACK^{®} cell culture chambers that occupied a cell growth area of 6360 cm² (Sigma, PA, US). Several umbilical cords were used; each gave rise to an individual MSC colony. All clones and corresponding EVs were characterized as described below.

Human bone marrow mesenchymal stem cells (BMSCs) were obtained from RoosterBio (RoosterBio, MD, US). Cells were cultured and expanded similarly to WJMSCs with the exception of using hMSC high performance media kit (RoosterBio, MD, US). Cells were maintained in an incubator at 37°C and 5% CO₂, Human foreskin (dermal) fibroblast cells (HDFs) were established by the tissue explant method (48). Freshly excised tissue was rinsed twice with phosphate buffer saline (PBS). Tissues sections were maintained in α-MEM supplemented with 10% FBS, 2 mM L-glutamine, and penicillin/streptomycin, and incubated for 7 days at 37°C in a humidified atmosphere of 5% CO₂. After 7 days, plastic adherent cell colonies were trypsinized and maintained in culture in αMEM, supplemented with 10% FBS, 2 mM L-glutamine, and penicillin/streptomycin. Cells were cultured and expanded similarly to WJMSCs (as described above). Cell counts were undertaken using trypan blue exclusion (1:1 v/v) and analyzed using a Cellometer Auto T4 (Nexcelom Biosciences, MA, USA).

### MSC Cell surface characterization and differentiation

Cytometric evaluation of cells surface profiles was carried out at passage 4. Antibodies used for cytometric analysis were obtained from BioLegend, CA, USA. and BD Biosciences, MA, US. WJMSCs and HDFs were selected for the expression of established MSC markers such as; APC/FITC-conjugated CD105, FITC-conjugated CD90, PE-conjugated CD44, and APC-conjugated CD73 and the absence of FITC-conjugated CD11b, PE-conjugated CD31, PE-conjugated CD34 and PE-conjugated CD45 using a set of fluorescently-conjugated antibodies **(****Figure 7****).** Flow cytometry was performed using a BD^{™} LSR II flow cytometer laser bench top flow cytometer, equipped with 407 nm, 488 nm and 640 nm lasers and BD FACS Diva software (v 5.0.3).

The differentiation potential of WJMSCs and HDF cultures to chondrocyte, osteocyte and adipocyte lineages was assessed using STEMPRO^{®}, Chondrogenesis, Osteogenesis and Adipogenesis differentiation kits respectively, (ThermoFisher Scientific), as per manufacturer's instructions **(****Figure 8****).**

### Transmission electron microscopy (TEM)

For EV visualization and morphological assessment, an aliquot from an EV preparation (5-10 µl) was adsorbed for 15 seconds to a formvar/carbon coated grid (Electron Microscopy Sciences, PA, US). Excess liquid was removed with Whatman Grade 1 filter paper (Sigma) followed by staining for 15 seconds with 2% uranyl acetate. Adsorbed EVs were examined on a JEOL 1200EX transmission electron microscope (TEM), and images were recorded with an AMT 2k CCD camera.

### Nanoparticle tracking analysis

Size and concentration distributions of EVs were determined using nanoparticle tracking analysis (NTA, NanoSight LM10 system, Malvern instruments, MA, US) as described previously (49). NTA is a laser illuminated microscopic technique equipped with a 642 nm laser and a high sensitivity digital camera system (OrcaFlash2.8, Hamamatsu, NanoSight Ltd) that determines the Brownian motion of nanoparticles in real-time to assess size and concentration. Samples were administered and recorded under controlled flow, using the NanoSight syringe pump and script control system, and for each sample, three videos of 60 seconds duration were recorded. Particle movement was analyzed using NTA software (version 3.0). Camera shutter speed was fixed at 30.01 ms and camera gain to 500. Camera sensitivity and detection threshold were (11-14) and(50-52), respectively. EV samples were diluted in EV-free dPBS. Samples were run in triplicate, from which EV distribution, size and mean concentration were calculated.

### Immunoblotting

Proteins in EV preparations were separated on a 4-20% polyacrylamide gel (Bio-Rad, Hercules, CA) and then transferred onto 0.45 µm PVDF membrane (Millipore, MA, US). Rabbit polyclonal anti-flotillin-1 and anti-CD63 antibodies (Santa Cruz Biotech, CA, US), and mouse polyclonal CD9 antibody (Santa Cruz Biotech, CA, US) were used for immunoblotting at dilutions recommended by the manufacturers.

### Experimental design

A schematic representation of the experimental design is highlighted in **Figure 2A****.** Newborn FVB mice were exposed to hyperoxia (HYRX, 75% O₂) for 7 days and were treated with or without MSC-EVs. As an additional control, NRMX-control animals received a single intravenous (IV) dose of WJMSC-exos. HYRX-exposed animals were compared to mice that remained at normoxia (NRMX, room air). At PN7 mice were sacrificed and the whole lung was harvested for flow cytometry analysis and total lung mRNA profiling. At two weeks of age (PN14), lung tissue was harvested for histology, total lung mRNA analysis, flow cytometry, and immunohistochemistry. Long-term outcomes were assessed at PN42. Here, mice were sacrificed for histology, pressure measurements, RV hypertrophy and pulmonary function tests. Animal experiments were approved by Boston Children's Hospital Animal Care and Use Committee.

### EV dosing

At PN4, EV preparations (50 µl of WJMSC-EVs, BMSC-EVs or HDF-EVs) were injected intravenously (IV) via the superficial temporal vein. EV preparations were diluted accordingly in dPBS to achieve an EV dose that corresponded to 0.5 × 10⁶ cell equivalents. Corresponding NTA and protein concentration values were recorded (Table 4).

**Table 4: EV-dosing. A 'gold-standard' method for EV quantification for EV-based therapeutics remains absent. At PN4, EV populations [corresponding fraction 9] were diluted accordingly to achieve a single bolus dose that equated to 0.5 × 10⁶ cell over 36 hrs. Exosome preparations were diluted with PBS to achieve this standard dosage in 50 µl per animal. Although exosome preparations from different cell types contained comparable nanoparticle numbers as assessed by NTA, their protein content varied. This is due to the presence of residual non exosomal protein contaminants still present in our highly purified density-flotation preparations. As a comparison, harvesting CM through differential centrifugation and pelleting at 100,000 xg, for 75 min yields preparations in which up to 95% of the protein represents fibronectin aggregates and other cell matrix detritus (our unpublished observations). Such pellets are oftentimes called exosomes, or extracellular vesicles, but there is little relation between protein concentration and nanoparticle numbers.**

| | **WJMSC-EVs** | **BMSC-EVs** | **HDF-EVs** |
|---|---|---|---|
| **Volume injected** | 50 µl | 50 µl | 50 µl |
| **Delivery route** | IV | IV | IV |
| **NTA (particles)** | 8.5 × 10⁸ | 7.2 × 10⁸ | 9.6 × 10⁸ |
| **Protein (µg)** | 0.9 µg | 3 µg | 1.5 µg |
| **Cell equivalent** | 0.5 × 10⁶ | 0.5 × 10⁶ | 0.5 × 10⁶ |

### Hyperoxia (HYRX) chamber

Neonatal pups were pooled and exposed to 75% O₂ in a plexiglass chamber or to room air beginning at PN1 and continuing for 7 days (PN1-7). Ventilation was adjusted by an Oxycycler controller (Biospherix, NY, US) to remove CO₂ so that it did not exceed 5,000 ppm (0.5%). Ammonia was removed by ventilation and charcoal filtration through an air purifier. Dams were rotated between room air and HYRX chambers every 48 hours to prevent excessive O₂ toxicity to the adult mice.

### Lung tissue perfusion, dissection and histology

Following anesthesia with 60 mg/kg pentobarbital (intraperitoneal (IP)), lungs were perfused with PBS through the right ventricle (RV) at a constant pressure of 25 cm H₂O. The left lung was carefully removed and stored at -80°C. The right lung was inflated to a fixed pressure of 15-20 cm H₂O with 4% paraformaldehyde (PFA) *in situ* and stored in 4% PFA overnight. Fixed lung tissue were transferred to 75% ethanol (EtOH) before subsequent processing, and paraffin embedding for sectioning as four distinct right lung lobes.

### Lung parenchymal, vascular morphometry, immunofluorescence, and immunohistochemistry

Following lung fixation with PFA (4% w/v), lung sections were analyzed for histology. Lung sections were stained with Hematoxylin and Eosin (H&E) and Masson's Trichrome (collagen deposition). Randomly selected areas (10-20 fields) from 5 µm thick lung sections were captured at 100× (H&E) and 200× (Masson's Trichrome) magnification using a Nikon Eclipse 80i microscope (Nikon, Tokyo, Japan). Calibrations for the images were done by acquiring standard micrometer images using the same magnification. Large airways and vessels were avoided for the lung morphometry. To measure mean linear intercept (MLI), a grid with parallel lines spaced at 58 µm was then overlaid onto the image, and the length of each chord, defined by the intercept with alveolar walls, was recorded using Metamorph software v.6.2r (Universal Imaging, Downingtown, PA). The degree of collagen deposition was measured using Metamorph software and expressed as percentage of collagen deposition per total septal area.

The loss of lung microvasculature was determined by counting the number of von Willebrand factor (vWF)-positive vessels in 8-12 random images at 100× magnification, stratified by diameter (<50 and 50-150 µm), using Metamorph software. Tissue sections were rehydrated and subjected to unmasking with 10mM citrate buffer, and then incubated with primary antibody (Dacko; polyclonal vWF Ab, 1:200). Afterwards, slides were washed and incubated with Alexa fluor 488-congugated donkey anti-rabbit (1:500) antibody.

To assess pulmonary vascularization, immunohistochemistry (IHC) analysis of alpha smooth muscle actin (α-SMA) was performed. Briefly, lung tissue sections were de-paraffinized in xylene and rehydrated. Tissue slides were treated with 0.3% H₂O₂ in methanol to inactivate endogenous peroxidases and blocked with horse serum for 20 min. After incubating with monoclonal anti-mouse α-SMA antibody (Sigma, MO, US) at a dilution of 1:125, secondary antibodies and peroxidase staining was applied according to manufacturer's instructions (Vector Laboratories, CA, US). The vessel wall thickness was assessed by measuring α-SMA positive staining in vessels <100 µm diameter in ~15 sections captured at 200× magnification. The wall thickness was measured using Metamorph software and compared between groups using the following equation: Medial wall thickness index (MTI) = 100 x (area_{[ext]} - area_{[int]}) / area_{[ext]} Area_{[ext]} and area_{[int]} denote the areas within the external and internal boundaries of the α-SMA layer, respectively. For histological analysis, investigators were blinded to experimental groups.

### Pulmonary function tests

To evaluate lung function, mice were anesthetized with a cocktail of ketamine (100 mg/kg), xylazine (6.5 mg/kg) and pentobarbital (20 mg/kg), administered via IP injection. Following tracheostomy, mice were mechanically ventilated at a rate of 150 breaths/minute, a tidal volume of 10 ml/kg, and a positive end-expiratory pressure (PEEP) of 3 cm H₂O with a computer-controlled small animal ventilator (Scireq, Montreal, Canada). Pressure-volume (PV)-loops, average airway resistance and lung compliance were determined, as previously described (50)

### Right ventricular systolic pressure (RVSP) and right ventricular hypertrophy (RVH) measurements

Direct right ventricular systolic pressure (RVSP) was measured as previously described (51). Briefly, mice were anesthetized with isoflurane in 100% O₂. A small incision was made in the abdominal wall, and the translucent diaphragm exposed. A 23-gauge butterfly needle with tubing attached to a pressure transducer was inserted through the diaphragm into the right ventricle and pressure measurements were recorded with PowerLab (ADInstruments, CO, US) monitoring hardware and software. Animals with heart rates <300 beats per minute were excluded. Mean RVSP over the first ten stable heartbeats was recorded. To quantify the degree of right ventricular hypertrophy (RVH), hearts and pulmonary vasculature were perfused *in situ* with PBS injection into the right ventricle (RV); hearts were excised and the RV to body weight (BW) (RV:BW) ratio were recorded. For Fulton's Index measurements (ratio of RV weight over left ventricle (LV) plus septal (S) weight, RV/[LV+S]). Whole hearts were weighed first, before the RV wall was dissected and weighted, followed by the remaining LV and ventricular septum. For physiological measurements, investigators were blinded to experimental groups.

### Lung RNA sequencing

Mice were harvested at PN7 and PN14 for lung RNA sequencing. Following perfusion through the RV with PBS, the left lung was removed and the large airways were dissected from the peripheral lung tissue. Whole lung RNA was isolated as previously described (52). RNA quality was verified using the Advanced Analytical Fragment Analyzer. Poly-adenylated mRNAs (1 µg) were isolated using oligoDT-based purification and used as input for reverse transcriptase reactions. Sequencing library preparation was then performed on cDNA using the TruSeq Stranded mRNA kit (Illumina, CA, US). The molar concentrations of the libraries were determined using the Advanced Analytical Fragment Analyzer, and the libraries were pooled at equimolar concentrations. Libraries were sequenced using a HiSeq 2500 instrument with High- Output single-read 50-base format. After removing short (<35 base pairs) or poor quality reads using Trimmomatic software (53), raw RNAseq data were aligned to a mouse reference genome (mm10 build) using STAR (54). Count tables for mouse annotated genes obtained from the UCSC Genome Browser were generated using feature Counts (55), and then differentially expressed genes were identified after normalizing datasets based on sequencing depth and running the Wald Test using the DESeq2 package in R (56). Using gene ontology (GO) analysis and Ingenuity Pathway Analysis (IPA), potential mRNA networks and targets were explored. RT-qPCR was used to validate the reproducibility of the sequencing results and levels of select candidate genes (as described below).

### Immunomodulatory potency of MSC-EVs: Macrophage polarization assay

Primary murine bone marrow-derived macrophages (BMDMs) were obtained by flushing the femur and tibia of 6-8-wk-old FVB mice, and culturing cells for 7 days in DMEM supplemented with 10% FBS, containing 30% v/v L929-conditioned medium (as a source of macrophage colony-stimulating factor; M-CSF). An alveolar macrophage (MH-S) cell line was obtained from ATCC (ATCC, US). MH-S cells or BMDMs were driven to M1 and M2 polarization states *in vitro.* Briefly, M1 polarization was initiated by lipopolysaccharide (LPS) 100 ng/ml and interferon-γ (IFNγ) 20 ng/ml stimulation. The M2 polarization state was driven by IL-4 (20 ng/ml) and IL-13 (20 ng/ml). Macrophage polarization (0.5 × 10⁶ BMDMs) was initiated with/without the presence of WJMSC-EV preparations (0.05-1 × 10⁶ cell equivalents) to evaluate the immunomodulatory functions of WJMSC-EVs. HDF-EVs served as a biologic and vehicle control. BMDM total RNA was isolated and gene expression levels were assessed by RT-qPCR as described in the supplemental methods. All cytokines were purchased from Peprotech (Peprotech, NJ, US).

To prove MSC-EVs interacted with macrophages, DiL-labeled EVs were derived from WJMSCs (5 × 10⁶) treated with 8 ml DiL (vibrant DiL labeling solution, Invitrogen, diluted 1:1000 in DMEM) for 30 minutes incubation at 37 °C and 5% CO₂. Post incubation, free tracer was removed by 2 × 10 ml PBS washes and maintained in 18 ml of EV-free SFM for 24 hours. After 24 hours, cell culture supernatant was taken and subjected to differential centrifugation steps (previously described) followed by ultra-centrifugation (100,000 × g for 70 min, 4 °C). Pelleted-EVs were washed in PBS by ultra-centrifugation (100,000 × g for 70 min, 4 °C). The supernatant from the last wash stage was kept and used as a control, to eliminate DiL contamination. MH-S cells grown to 75% confluence were then treated with DiL-labeled MSC-EVs for 3 hours (37 °C and 5% CO₂). To remove non-adherent MSC-EVs, 3 × 10 ml PBS washes were performed. Cells and adhered EVs were maintained in growth medium until visualized by fluorescence using a Nikon Eclipse 80i microscope (Nikon, Tokyo, Japan).

### Cytometric analysis of mouse whole lung

Flow cytometry was used to assess lung cell population concentrations and lung macrophage phenotypes, as previously described (57). Mice were harvested at PN7 and PN14. Following perfusion through the RV with PBS, the left lung was removed and the large airways were dissected from the peripheral lung tissue. Lung tissue was cut into small pieces and transferred into falcon tubes (Fisher Scientific, NH, US), and processed in 5 ml digestion buffer consisting of RPMI-1640 (Invitrogen, CA, US), Collagenase IV (1.6 mg/ml); and DNAse1 (50 unit/ml), both from Worthington Biochemical Corp, NJ, US. IIomogcnized lungs were passed through 40- µm cell strainer (Corning, MA, US) to obtain a single-cell suspension. The remaining red blood cells were lysed using red blood cell lysis buffer (Roche, IN, US). The cell suspension was stained with antibodies; PE/Cy7 conjugated-Cd45, FITC conjugated-Cd11b, PerCP Cy 5.5 conjugated-Cd11c, and PE-conjugated Cd64. Macrophage phenotype markers Cd40, Cd86 and Cd206 were conjugated to brilliant violet 421 (BV421). All antibodies were obtained from Biolegend or BD Biosciences. Data were acquired on a BD LSR II flow cytometer using BD FACSDiva software (BD Biosciences, MA, US). Compensation was adjusted accordingly and supported by UltraComp ebeads (Affymetrix, CA, US). Fluorescence-minus-one controls were used accordingly. Cell populations were identified using sequential gating strategy and quantified using CountBright^{™} Absolute Counting Beads (ThermoFisher, MA, US) and recorded as either an absolute concentration or as a percentage of the total cell population. The expression of macrophage activation markers is presented as median fluorescence intensity (MFI).

### Reverse Transcription-Polymerase Chain Reaction Analysis

For lung mRNA assessment, following perfusion through the RV with sterile PBS, the left lung was removed for RNA extraction, as previously described (51, 52) and subsequent mRNA analysis by RT-qPCR. TRIZOL (ThermoFisher) was used to extract BMDM total RNA, as per manufacturer's instructions. TAQMAN^{®} primers used in the PCR reactions including TNFα, Ccl2, Ccl7, Il6, Il33, Cd206, Retnla, and Arg1 were obtained from Invitrogen. Nup133 served as an internal standard. Analysis of the fold change was performed as previously described (51).

### REFERENCES

1. Jobe AH, Bancalari E. Bronchopulmonary dysplasia. Am J Respir Crit Care Med 2001;163:1723-1729.
2. Stenmark KR, Abman SH. Lung vascular development: Implications for the pathogenesis of bronchopulmonary dysplasia. Annual review of physiology 2005;67:623-661.
3. Baraldi E, Filippone M. Chronic lung disease after premature birth. New England Journal of Medicine 2007;357:1946-1955.
4. Khemani E, McElhinney DB, Rhein L, Andrade O, Lacro RV, Thomas KC, Mullen MP. Pulmonary artery hypertension in formerly premature infants with bronchopulmonary dysplasia: Clinical features and outcomes in the surfactant era. Pediatrics 2007;120:1260-1269.
5. del Cerro MJ, Sabaté Rotes A, Carton A, Deiros L, Bret M, Cordeiro M, Verdú C, Barrios MI, Albajara L, Gutierrez-Larraya F. Pulmonary hypertension in bronchopulmonary dysplasia: Clinical findings, cardiovascular anomalies and outcomes. Pediatric Pulmonology 2014;49:49-59.
6. Borghesi A, Cova C, Gazzolo D, Stronati M. Stem cell therapy for neonatal diseases associated with preterm birth. Journal of Clinical Neonatology 2013;2:1-7.
7. Borghesi A, Massa M, Campanelli R, Bollani L, Tzialla C, Figar TA, Ferrari G, Bonetti E, Chiesa G, de Silvestri A, Spinillo A, Rosti V, Stronati M. Circulating endothelial progenitor cells in preterm infants with bronchopulmonary dysplasia. American Journal of Respiratory and Critical Care Medicine 2009;180:540-546.
8. Baker CD, Balasubramaniam V, Mourani PM, Sontag MK, Black CP, Ryan SL, Abman SH. Cord blood angiogenic progenitor cells are decreased in bronchopulmonary dysplasia. European Respiratory Journal 2012;40:1516-1522.
9. Aslam M, Baveja R, Liang OD, Fernandez-Gonzalez A, Lee C, Mitsialis SA, Kourembanas S. Bone marrow stromal cells attenuate lung injury in a murine model of neonatal chronic lung disease. American Journal of Respiratory and Critical Care Medicine 2009;180:1122-1130.
10. van Haaften T, Byrne R, Bonnet S, Rochefort GY, Akabutu J, Bouchentouf M, Rey-Parra GJ, Galipeau J, Haromy A, Eaton F, Chen M, Hashimoto K, Abley D, Korbutt G, Archer SL, Thébaud B. Airway delivery of mesenchymal stem cells prevents arrested alveolar growth in neonatal lung injury in rats. American Journal of Respiratory and Critical Care Medicine 2009;180:1131-1142.
11. Zhang X, Wang H, Shi Y, Peng W, Zhang S, Zhang W, Xu J, Mei Y, Feng Z. Role of bone marrow-derived mesenchymal stem cells in the prevention of hyperoxia-induced lung injury in newborn mice. Cell Biology International 2012;36:589-594.
12. Baker CD, Seedorf GJ, Wisniewski BL, Black CP, Ryan SL, Balasubramaniam V, Abman SH. Endothelial colony-forming cell conditioned media promote angiogenesis in vitro and prevent pulmonary hypertension in experimental bronchopulmonary dysplasia. American Journal of Physiology - Lung Cellular and Molecular Physiology 2013;305:L73-L81.
13. Hodges RJ, Jenkin G, Hooper SB, Allison B, Lim R, Dickinson H, Miller SL, Vosdoganes P, Wallace EM. Human amnion epithelial cells reduce ventilation-induced preterm lung injury in fetal sheep. American Journal of Obstetrics and Gynecology 2012;206:448.e448- 448.e415.
14. Kourembanas S. Expanding the pool of stem cell therapy for lung growth and repair. Circulation 2014;129:2091-2093.
15. Kourembanas S. Stem cell-based therapy for newborn lung and brain injury: Feasible, safe, and the next therapeutic breakthrough? The Journal of pediatrics 2014;164:954-956.
16. Lee JW, Fang X, Krasnodembskaya A, Howard JP, Matthay MA. Concise review: Mesenchymal stem cells for acute lung injury: Role of paracrine soluble factors. Stem cells 2011;29:913-919.
17. Liang OD, Mitsialis SA, Chang MS, Vergadi E, Lee C, Aslam M, Fernandez-Gonzalez A, Liu X, Baveja R, Kourembanas S. Mesenchymal stromal cells expressing heme oxygenase-1 reverse pulmonary hypertension. Stem cells 2011;29:99-107.
18. Hansmann G, Fernandez-Gonzalez A, Aslam M, Vitali SH, Martin T, Mitsialis SA, Kourembanas S. Mesenchymal stem cell-mediated reversal of bronchopulmonary dysplasia and associated pulmonary hypertension. Pulmonary Circulation 2012;2:170-181.
19. Curley GF, Hayes M, Ansari B, Shaw G, Ryan A, Barry F, O'Brien T, O'Toole D, Laffey JG. Mesenchymal stem cells enhance recovery and repair following ventilator-induced lung injury in the rat. Thorax 2012;67:496-501.
20. Pierro M, Ionescu L, Montemurro T, Vadivel A, Weissmann G, Oudit G, Emery D, Bodiga S, Eaton F, Péault B, Mosca F, Lazzari L, Thébaud B. Short-term, long-term and paracrine effect of human umbilical cord-derived stem cells in lung injury prevention and repair in experimental bronchopulmonary dysplasia. Thorax 2013;68:475-484.
21. Lee C, Mitsialis SA, Aslam M, Vitali SH, Vergadi E, Konstantinou G, Sdrimas K, Fernandez-Gonzalez A, Kourembanas S. Exosomes mediate the cytoprotective action of mesenchymal stromal cells on hypoxia-induced pulmonary hypertension. Circulation 2012;126:2601-2611.
22. Cruz FF, Borg ZD, Goodwin M, Sokocevic D, Wagner DE, Coffey A, Antunes M, Robinson KL, Mitsialis SA, Kourembanas S, Thane K, Hoffman AM, McKenna DH, Rocco PR, Weiss DJ. Systemic administration of human bone marrow-derived mesenchymal stromal cell extracellular vesicles ameliorates aspergillus hyphal extract-induced allergic airway inflammation in immunocompetent mice. Stem Cells Transl Med 2015;4:1302-1316.
23. Sdrimas K, Kourembanas S. Msc microvesicles for the treatment of lung disease: A new paradigm for cell-free therapy. Antioxidants & redox signaling 2014;21:1905-1915.
24. Arslan F, Lai RC, Smeets MB, Akeroyd L, Choo A, Aguor ENE, Timmers L, van Rijen HV, Doevendans PA, Pasterkamp G, Lim SK, de Kleijn DP. Mesenchymal stem cell-derived exosomes increase atp levels, decrease oxidative stress and activate pi3k/akt pathway to enhance myocardial viability and prevent adverse remodeling after myocardial ischemia/reperfusion injury. Stem Cell Research 2013;10:301-312.
25. Lai RC, Arslan F, Lee MM, Sze NSK, Choo A, Chen TS, Salto-Tellez M, Timmers L, Lee CN, El Oakley RM, Pasterkamp G, de Kleijn DPV, Lim SK. Exosome secreted by msc reduces myocardial ischemia/reperfusion injury. Stem Cell Research 2010;4:214-222.
26. Zhang G, Wang D, Miao S, Zou X, Liu G, Zhu Y. Extracellular vesicles derived from mesenchymal stromal cells may possess increased therapeutic potential for acute kidney injury compared with conditioned medium in rodent models: A meta-analysis. Experimental and Therapeutic Medicine 2016;11:1519-1525.
27. Dorronsoro A, Robbins PD. Regenerating the injured kidney with human umbilical cord mesenchymal stem cell-derived exosomes. Stem Cell Research & Therapy 2013;4:39-39.
28. Burrello J, Monticone S, Gai C, Gomez Y, Kholia S, Camussi G. Stem cell-derived extracellular vesicles and immune-modulation. Frontiers in Cell and Developmental Biology 2016;4:83.
29. Zhang B, Yin Y, Lai RC, Tan SS, Choo ABH, Lim SK. Mesenchymal stem cells secrete immunologically active exosomes. Stem Cells and Development 2013;23:1233-1244.
30. Xin H, Li Y, Buller B, Katakowski M, zhang Y, Wang X, Shang X, Zhang ZG, Chopp M. Exosome-mediated transfer of mir-133b from multipotent mesenchymal stromal cells to neural cells contributes to neurite outgrowth. Stem cells (Dayton, Ohio) 2012;30:1556-1564.
31. Kalani A, Tyagi N. Exosomes in neurological disease, neuroprotection, repair and therapeutics: Problems and perspectives. Neural Regeneration Research 2015;10:1565-1567.
32. Kourembanas S. Exosomes: Vehicles of intercellular signaling, biomarkers, and vectors of cell therapy. Annual review of physiology 2015;77:13-27.
33. Linares R, Tan S, Gounou C, Arraud N, Brisson AR. High-speed centrifugation induces aggregation of extracellular vesicles. 2015 2015.
34. Suki B, Sato S, Parameswaran H, Szabari MV, Takahashi A, Bartolák-Suki E. Emphysema and mechanical stress-induced lung remodeling. Physiology 2013;28:404-413.
35. Vergadi E, Chang MS, Lee C, Liang OD, Liu X, Fernandez-Gonzalez A, Mitsialis SA, Kourembanas S. Early macrophage recruitment and alternative activation are critical for the later development of hypoxia-induced pulmonary hypertension. Circulation 2011;123:1986-1995.
36. Schmalisch G, Wilitzki S, Roehr CC, Proquitté H, Biihrer C. Development of lung function in very low birth weight infants with or without bronchopulmonary dysplasia: Longitudinal assessment during the first 15 months of corrected age. BMC Pediatrics 2012;12:37.
37. Johnston LK, Rims CR, Gill SE, McGuire JK, Manicone AM. Pulmonary macrophage subpopulations in the induction and resolution of acute lung injury. American journal of respiratory cell and molecular biology 2012;47:417-426.
38. Laskin DL, Sunil VR, Gardner CR, Laskin JD. Macrophages and tissue injury: Agents of defense or destruction? Annual review of pharmacology and toxicology 2011;51:267-288.
39. Speer CP. Pulmonary inflammation and bronchopulmonary dysplasia. J Perinatol 2006;26 Suppl 1:S57-62; discussion S63-54.
40. Abreu SC, Weiss DJ, Rocco PRM. Extracellular vesicles derived from mesenchymal stromal cells: A therapeutic option in respiratory diseases? Stem Cell Research & Therapy 2016;7:53.
41. Vasandan AB, Jahnavi S, Shashank C, Prasad P, Kumar A, Prasanna SJ. Human mesenchymal stem cells program macrophage plasticity by altering their metabolic status via a pge2-dependent mechanism. Scientific Reports 2016;6:38308.
42. Lo Sicco C, Reverberi D, Balbi C, Ulivi V, Principi E, Pascucci L, Becherini P, Bosco MC, Varesio L, Franzin C, Pozzobon M, Cancedda R, Tasso R. Mesenchymal stem cell-derived extracellular vesicles as mediators of anti-inflammatory effects: Endorsement of macrophage polarization. Stem Cells Translational Medicine 2017;6:1018-1028.
43. Bhandari V. Postnatal inflammation in the pathogenesis of bronchopulmonary dysplasia. Birth defects research Part A, Clinical and molecular teratology 2014;100:189-201.
44. Viscardi RM. Perinatal inflammation and lung injury. Seminars in fetal & neonatal medicine 2012;17:30-35.
45. Lawrence T, Natoli G. Transcriptional regulation of macrophage polarization: Enabling diversity with identity. Nature reviews Immunology 2011;11:750-761.
46. Mitsialis SA, Kourembanas S. Stem cell-based therapies for the newborn lung and brain: Possibilities and challenges. Seminars in perinatology 2016;40:138-151.
47. Pirjali T, Azarpira N, Ayatollahi M, Aghdaie MH, Geramizadeh B, Talai T. Isolation and Characterization of Human Mesenchymal Stem Cells Derived from Human Umbilical Cord Wharton's Jelly and Amniotic Membrane. International Journal of Organ Transplantation Medicine. 2013;4(3):111-6.
48. Nichols W, Murphy D, Cristofalo V, Toji L, Greene A, Dwight S. Characterization of a new human diploid cell strain, IMR-90. Science. 1977;196(4285):60-3.
49. Willis GR, Connolly K, Ladell K, Davies TS, Guschina IA, Ramji D, et al. Young women with polycystic ovary syndrome have raised levels of circulating annexin V-positive platelet microparticles. Human Reproduction. 2014;29(12):2756-63.
50. Wang R, Lu B, Gerard C, Gerard NP. C5L2, the Second C5a Anaphylatoxin Receptor, Suppresses LPS-Induced Acute Lung Injury. Am J Respir Cell Mol Biol. 2016;55(5):657-66. Epub 2016/11/01.
51. Lee C, Mitsialis SA, Aslam M, Vitali SH, Vergadi E, Konstantinou G, et al. Exosomes Mediate the Cytoprotective Action of Mesenchymal Stromal Cells on Hypoxia-Induced Pulmonary Hypertension. Circulation. 2012;126(22):2601-11.
52. Chomczynski P, Sacchi N. Single-step method of RNA isolation by acid guanidinium thiocyanate-phenol-chloroform extraction. Analytical Biochemistry. 1987;162(1):156-9.
53. Bolger AM, Lohse M, Usadel B. Trimmomatic: a flexible trimmer for Illumina sequence data. Bioinformatics. 2014;30(15):2114-20.
54. Dobin A, Davis CA, Schlesinger F, Drenkow J, Zaleski C, Jha S, et al. STAR: ultrafast universal RNA-seq aligner. Bioinformatics. 2012;29(1): 15-21.
55. Liao Y, Smyth GK, Shi W. featureCounts: an efficient general purpose program for assigning sequence reads to genomic features. Bioinformatics. 2013;30(7):923-30.
56. Love MI, Huber W, Anders S. Moderated estimation of fold change and dispersion for RNA-seq data with DESeq2. Genome Biology. 2014;15(12):550.
57. Misharin AV, Morales-Nebreda L, Mutlu GM, Budinger GRS, Perlman H. Flow Cytometric Analysis of Macrophages and Dendritic Cell Subsets in the Mouse Lung. American Journal of Respiratory Cell and Molecular Biology. 2013;49(4):503-10.

### EQUIVALENTS AND SCOPE

Those skilled in the art will recognize, or be able to ascertain using no more than routine experimentation, many equivalents of the embodiments described herein. The scope of the present disclosure is not intended to be limited to the above description, but rather is as set forth in the appended claims.

Articles such as "a," "an," and "the" may mean one or more than one unless indicated to the contrary or otherwise evident from the context. Claims or descriptions that include "or" between two or more members of a group are considered satisfied if one, more than one, or all of the group members are present, unless indicated to the contrary or otherwise evident from the context. The disclosure of a group that includes "or" between two or more group members provides embodiments in which exactly one member of the group is present, embodiments in which more than one members of the group are present, and embodiments in which all of the group members are present. For purposes of brevity those embodiments have not been individually spelled out herein, but it will be understood that each of these embodiments is provided herein and may be specifically claimed or disclaimed.

It is to be understood that the disclosure encompasses all variations, combinations, and permutations in which one or more limitation, element, clause, or descriptive term, from one or more of the claims or from one or more relevant portion of the description, is introduced into another claim. For example, a claim that is dependent on another claim can be modified to include one or more of the limitations found in any other claim that is dependent on the same base claim. Furthermore, where the claims recite a composition, it is to be understood that methods of making or using the composition according to any of the methods of making or using disclosed herein or according to methods known in the art, if any, are included, unless otherwise indicated or unless it would be evident to one of ordinary skill in the art that a contradiction or inconsistency would arise.

Where elements are presented as lists, e.g., in Markush group format, it is to be understood that every possible subgroup of the elements is also disclosed, and that any element or subgroup of elements can be removed from the group. It is also noted that the term "comprising" is intended to be open and permits the inclusion of additional elements or steps. It should be understood that, in general, where an embodiment, product, or method is referred to as comprising particular elements, features, or steps, embodiments, products, or methods that consist, or consist essentially of, such elements, features, or steps, are provided as well. For purposes of brevity those embodiments have not been individually spelled out herein, but it will be understood that each of these embodiments is provided herein and may be specifically claimed or disclaimed.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in some embodiments, to the tenth of the unit of the lower limit of the range, unless the context clearly dictates otherwise. For purposes of brevity, the values in each range have not been individually spelled out herein, but it will be understood that each of these values is provided herein and may be specifically claimed or disclaimed. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

Where websites are provided, URL addresses are provided as non-browser-executable codes, with periods of the respective web address in parentheses. The actual web addresses do not contain the parentheses.

In addition, it is to be understood that any particular embodiment of the present disclosure may be explicitly excluded from any one or more of the claims. Where ranges are given, any value within the range may explicitly be excluded from any one or more of the claims. Any embodiment, element, feature, application, or aspect of the compositions and/or methods of the disclosure, can be excluded from any one or more claims. For purposes of brevity, all of the embodiments in which one or more elements, features, purposes, or aspects is excluded are not set forth explicitly herein.

## Claims

1. A pharmaceutical composition for treating hyperoxia-induced lung injury, hyperoxia-induced pulmonary hypertension, bronchopulmonary dysplasia, and reducing pulmonary fibrosis, comprising an isolated mesenchymal stem cell (MCS) exosome, wherein:
(i) the isolated MSC exosome comprises four or more markers selected from the group consisting of FLT1, PLAUR, MME, CDH2, SLC16A3, CDH13, ITGB5, ITGA11, APP, GPC6, IGSF8, IRP2, TSPAN9, DAG1, SLC38A2, SLC12A8, GJA1, ITGA1, PLXNB2, SLC16A1, and PROCR;
(ii) the isolated MSC exosome comprises one or more markers that is enriched compared to a fibroblast exosome, the one or more markers selected from the group consisting of: PTk7, CD109, SLC1A5, ITGA3, ITGA2, BSG, DPP4, ATP1A1, FAP, VASN, IGF2R, and CD82; and/or
(iii) the isolated MSC exosome comprises one or more markers that is enriched compared to a fibroblast exosome, the one or more markers selected from the group consisting of RCN1, RAP1B, GDF15, FLT1, OLFML3, PLOD2, PSMA4, PAPPA, INHBA, SPOCK1, HSPB1, LOXL4, POLD3, CALU, IGFBP4, C4B, TINAGL1, SULF1, TPM3, AHNAK, CALR, RRAS2, PFKP, and COL16A1,
wherein the isolated mesenchymal stem cell (MCS) exosome is obtained by a method comprising:
a) obtaining MSC-CM from WJMSCs, BMSCs or human dermal fibroblasts (HDFs) cultures;
b) concentrating the MSC-CM;
c) fractionating the concentrated MSC-CM using an iodixanol (IDX) cushion gradient; wherein the fractionating comprises floating the MSC-CM on the IDX cushion gradient and ultracentrifugation;
d) collecting a fraction that floats in the gradient at a density of ~1.18g/ml; and
e) extracting exosomes from the collected fraction;
thereby isolating the mesenchymal stem cell (MCS) exosome.

2. The pharmaceutical composition of claim 1, wherein the isolated MSC exosome further comprises one or more markers selected from the group consisting of: FLOT1, CD9, and CD63.

3. The pharmaceutical composition of any one of claims 1-2, wherein the isolated MSC exosome:
(i) is isolated from MSC-conditioned media;
(ii) is from Warton's Jelly or bone marrow;
(iii) is substantially free of non-exosomal protein contaminants;
(iv) has a diameter of about 30-150 nm; and / or
(v) has immunomodulatory activity.

4. The pharmaceutical composition of any one of claims 1-3, further comprising
i) a secondary agent and / or
ii) a pharmaceutically acceptable carrier.

5. The pharmaceutical composition of claim 4, wherein the secondary agent is a pulmonary surfactant, a steroid, an antioxidant, or inhaled nitric oxide.

6. The pharmaceutical composition of any one of claims 1-5, for use in treating hyperoxia-induced lung injury, hyperoxia-induced pulmonary hypertension, bronchopulmonary dysplasia, and reducing pulmonary fibrosis in a subject.

7. The pharmaceutical composition for use according to claim 6, wherein the subject is a human subject.

8. The pharmaceutical composition for use according to claim 7, wherein:
i) the human subject was born before 37 weeks of gestation or was born before 26 weeks of gestation;
ii) the subject has hyperoxia-induced lung injury;
iii) the subject has been administered oxygen or has been on a ventilator;
iv) the subject has or is at risk of developing bronchopulmonary dysplasia (BPD);
v) the subject has hyperoxia-induced pulmonary hypertension;
vi) the subject exhibits peripheral pulmonary arterial remodeling;
vii) the subject has inflammation in the lung; and / or
viii) the subject is less than four weeks of age, four weeks to 3 years of age, 3-18 years of age; or is an adult.

9. The pharmaceutical composition for use according to claim 7 or 8, wherein:
i) the isolated MSC exosome is administered in a bolus dose to the subject or wherein the isolated MSC exosome is administered repeatedly to the subject;
ii) wherein the isolated MSC exosome improves lung function;
iii) wherein the isolated MSC exosome restores lung architecture;
v) wherein the isolated MSC exosome reduces inflammation in the lung; and / or
vi) wherein the isolated MSC exosome reduces peripheral pulmonary arterial remodeling.

10. The pharmaceutical composition for use according to claim 9, wherein the isolated MSC exosome is administered within an hour of birth or wherein the isolated MSC exosome is administered within one month of birth.

11. The pharmaceutical composition for use according to any one of claims 7 - 10, wherein:
i) the isolated MSC exosome is administered intravenously or intranasally;
ii) the isolated MSC exosome is administered to the lung or trachea of the subject;
iii) the isolated MSC exosome is administered by inhalation;
iv) the isolated MSC exosome is administered in an aerosol;
v) the isolated MSC exosome is administered using a nebulizer; or
vi) the isolated MSC exosome is administered using an intratracheal tube.

12. The pharmaceutical composition for use according to claim 6, wherein the subject is a mammal.

13. The pharmaceutical composition for use according to claim 12
i) wherein the mammal is a rodent, further optionally wherein the rodent is a mouse or a rat.

14. A method of isolating a mesenchymal stem cell (MSC) exosome, the method comprising fractionating MSC-conditioned media using an iodixanol (IDX) cushion gradient and collecting a fraction that is substantially free of non-exosomal protein contaminants according to the method **characterized in** claim 1.

15. The method of claim 14, wherein the MSC-conditioned media is concentrated prior to fractionation.

16. The method according to claim 15 wherein the MSC-conditioned media is concentrated via tangential flow filtration.

17. The method of any one of claims 14-16, wherein the fractionating comprises floating the MSC-conditioned media on the IDX cushion gradient and ultracentrifugation.

## Patentansprüche

1. Pharmazeutische Zusammensetzung zur Behandlung von hyperoxiebedingten Lungenverletzungen, hyperoxiebedingter pulmonaler Hypertonie, bronchopulmonaler Dysplasie und zur Verringerung von Lungenfibrose, umfassend ein isoliertes mesenchymales Stammzell-Exosom (MCS), wobei:
(i) das isolierte MSC-Exosom vier oder mehr Marker umfasst, ausgewählt aus der Gruppe bestehend aus FLT1, PLAUR, MME, CDH2, SLC16A3, CDH13, ITGB5, ITGA11, APP, GPC6, IGSF8, IRP2, TSPAN9, DAG1, SLC38A2, SLC12A8, GJA1, ITGA1, PLXNB2, SLC16A1 und PROCR;
(ii) das isolierte MSC-Exosom einen oder mehrere Marker umfasst, die im Vergleich zu einem Fibroblasten-Exosom angereichert sind, wobei der eine oder die mehreren Marker ausgewählt sind aus der Gruppe bestehend aus PTk7, CD109, SLC1A5, ITGA3, ITGA2, BSG, DPP4, ATP1A1, FAP, VASN, IGF2R und CD82; und/oder
(iii) das isolierte MSC-Exosom einen oder mehrere Marker umfasst, die im Vergleich zu einem Fibroblasten-Exosom angereichert sind, wobei der eine oder die mehreren Marker ausgewählt sind aus der Gruppe bestehend aus RCN1, RAP1B, GDF15, FLT1, OLFML3, PLOD2, PSMA4, PAPPA, INHBA, SPOCK1, HSPB1, LOXL4, POLD3, CALU, IGFBP4, C4B, TINAGL1, SULF1, TPM3, AHNAK, CALR, RRAS2, PFKP und COL16A1,
wobei das isolierte mesenchymale Stammzell (MCS)-Exosom durch ein Verfahren erhalten wird, umfassend:
a) Erhalten von MSC-CM aus WJMSCs, BMSCs oder menschlichen Hautfibroblasten (HDFs)-Kulturen;
b) Konzentrieren des MSC-CM;
c) Fraktionieren des konzentrierten MSC-CM unter Verwendung eines lodixanol (IDX)-Gradienten; wobei das Fraktionieren das Schwimmenlassen des MSC-CM auf dem IDX-Gradienten und Ultrazentrifugieren umfasst;
d) Sammeln einer Fraktion, die im Gradienten bei einer Dichte von ~1,18 g/ml schwimmt; und
e) Extrahieren von Exosomen aus der gesammelten Fraktion;
wodurch mesenchymale Stammzell (MCS)-Exosom isoliert wird.

2. Pharmazeutische Zusammensetzung nach Anspruch 1, wobei das isolierte MSC-Exosom ferner einen oder mehrere Marker umfasst, ausgewählt aus der Gruppe bestehend aus FLOT1, CD9 und CD63.

3. Pharmazeutische Zusammensetzung nach einem der Ansprüche 1-2, wobei das isolierte MSC-Exosom:
(i) aus MSC-konditionierten Medien isoliert ist;
(ii) aus Warton's Jelly oder Knochenmark stammt;
(iii) im Wesentlichen frei von nicht-exosomalen Proteinverunreinigungen ist;
(iv) einen Durchmesser von etwa 30-150 nm aufweist; und/oder
(v) immunmodulatorische Aktivität aufweist.

4. Pharmazeutische Zusammensetzung nach einem der Ansprüche 1-3, ferner umfassend:
i) einen sekundären Wirkstoff und/oder
ii) einen pharmazeutisch akzeptablen Träger.

5. Pharmazeutische Zusammensetzung nach Anspruch 4, wobei der sekundäre Wirkstoff ein Lungensurfaktant, ein Steroid, ein Antioxidans oder inhaliertes Stickstoffmonoxid ist.

6. Pharmazeutische Zusammensetzung nach einem der Ansprüche 1 bis 5 zur Verwendung bei der Behandlung von hyperoxiebedingten Lungenverletzungen, hyperoxiebedingter pulmonaler Hypertonie, bronchopulmonaler Dysplasie und zur Verringerung von Lungenfibrose bei einem Subjekt.

7. Pharmazeutische Zusammensetzung zur Verwendung nach Anspruch 6, wobei das Subjekt ein Mensch ist.

8. Pharmazeutische Zusammensetzung zur Verwendung nach Anspruch 7, wobei:
i) der Mensch vor der 37. Schwangerschaftswoche oder vor der 26. Schwangerschaftswoche geboren wurde;
ii) das Subjekt eine hyperoxiebedingte Lungenverletzung hat;
iii) dem Subjekt Sauerstoff verabreicht wurde oder er beatmet wurde;
iv) das Subjekt an bronchopulmonaler Dysplasie (BPD) leidet oder ein Risiko dafür aufweist;
v) das Subjekt an einer hyperoxiebedingten pulmonalen Hypertonie leidet;
vi) das Subjekt eine periphere pulmonale arterielle Remodellierung aufweist;
vii) das Subjekt eine Entzündung in der Lunge aufweist; und/oder
viii) das Subjekt weniger als vier Wochen alt ist, vier Wochen bis drei Jahre alt ist, drei bis 18 Jahre alt ist oder ein Erwachsener ist.

9. Pharmazeutische Zusammensetzung zur Verwendung nach Anspruch 7 oder 8, wobei:
i) das isolierte MSC-Exosom dem Subjekt in einer Bolusdosis verabreicht wird oder wobei das isolierte MSC-Exosom dem Subjekt wiederholt verabreicht wird;
ii) wobei das isolierte MSC-Exosom die Lungenfunktion verbessert;
iii) wobei das isolierte MSC-Exosom die Lungenarchitektur wiederherstellt;
v) wobei das isolierte MSC-Exosom Entzündungen in der Lunge reduziert; und/oder
vi) wobei das isolierte MSC-Exosom die periphere pulmonale arterielle Umgestaltung reduziert.

10. Pharmazeutische Zusammensetzung zur Verwendung nach Anspruch 9, wobei das isolierte MSC-Exosom innerhalb einer Stunde nach der Geburt verabreicht wird oder wobei das isolierte MSC-Exosom innerhalb eines Monats nach der Geburt verabreicht wird.

11. Pharmazeutische Zusammensetzung zur Verwendung nach einem der Ansprüche 7 bis 10, wobei:
i) das isolierte MSC-Exosom intravenös oder intranasal verabreicht wird;
ii) das isolierte MSC-Exosom in die Lunge oder Luftröhre des Subjekts verabreicht wird;
iii) das isolierte MSC-Exosom durch Inhalation verabreicht wird;
iv) das isolierte MSC-Exosom in einem Aerosol verabreicht wird;
v) das isolierte MSC-Exosom unter Verwendung eines Verneblers verabreicht wird; oder
vi) das isolierte MSC-Exosom unter Verwendung eines intratrachealen Tubus verabreicht wird.

12. Pharmazeutische Zusammensetzung zur Verwendung nach Anspruch 6, wobei das Subjekt ein Säugetier ist.

13. Pharmazeutische Zusammensetzung zur Verwendung gemäß Anspruch 12,
i) wobei das Säugetier ein Nagetier ist, wobei das Nagetier optional eine Maus oder eine Ratte ist.

14. Verfahren zur Isolierung eines mesenchymalen Stammzell (MSC)-Exosoms, wobei das Verfahren die Fraktionierung von MSC-konditionierten Medien unter Verwendung eines lodixanol (IDX)-Gradienten und das Sammeln einer Fraktion umfasst, die im Wesentlichen frei von nicht-exosomalen Proteinverunreinigungen ist, gemäß dem in Anspruch 1 charakterisierten Verfahren.

15. Verfahren nach Anspruch 14, wobei die MSC-konditionierten Medien vor der Fraktionierung konzentriert werden.

16. Verfahren nach Anspruch 15, wobei das MSC-konditionierte Medium durch Tangentialflussfiltration konzentriert wird.

17. Verfahren nach einem der Ansprüche 14 bis 16, wobei die Fraktionierung das Schwimmenlassen des MSC-konditionierten Mediums auf dem IDX-Gradienten und die Ultrazentrifugation umfasst.

## Revendications

1. - Composition pharmaceutique pour traiter une lésion pulmonaire induite par hyperoxie, l'hypertension pulmonaire induite par hyperoxie, la dysplasie broncho-pulmonaire et pour réduire la fibrose pulmonaire, comprenant un exosome isolé à partir de cellules souches mésenchymateuses (CSM), dans laquelle :
(i) l'exosome isolé à partir de CSM comprend au moins quatre marqueurs choisis dans le groupe consistant en FLT1, PLAUR, MME, CDH2, SLC16A3, CDH13, ITGB5, ITGA11, APP, GPC6, IGSF8, IRP2, TSPAN9, DAG1, SLC38A2, SLC12A8, GJA1, ITGA1, PLXNB2, SLC16A1 et PROCR ;
(ii) l'exosome isolé à partir de CSM comprend au moins un marqueur qui est enrichi par comparaison à un exosome de fibroblaste, le ou les marqueurs étant choisis dans le groupe consistant en : PTk7, CD109, SLC1A5, ITGA3, ITGA2, BSG, DPP4, ATPlAl, FAP, VASN, IGF2R et CD82 ; et/ou
(iii) l'exosome isolé à partir de CSM comprend au moins marqueur qui est enrichi par comparaison à un exosome de fibroblaste, le ou les marqueurs étant choisis dans le groupe consistant en RCN1, RAP1B, GDF15, FLT1, OLFML3, PLOD2, PSMA4, PAPPA, INHBA, SPOCK1, HSPB1, LOXL4, POLD3, CALU, IGFBP4, C4B, TINAGL1, SULF1, TPM3, AHNAK, CALR, RRAS2, PFKP et COL16A1,
dans laquelle l'exosome isolé à partir de cellules souches mésenchymateuses (CSM) est obtenu par un procédé comprenant :
a) obtenir un milieu conditionné de CSM (MC-CSM) à partir de cultures de WJMSC, de BMSC ou de fibroblastes dermiques humains (HDF) ;
b) concentrer le MC-CSM ;
c) fractionner le MC-CSM concentré à l'aide d'un gradient coussin d'iodixanol (IDX), le fractionnement comprenant une flottaison du CM-CSM sur le gradient coussin d'IDX et une ultracentrifugation ;
d) collecter une fraction qui flotte dans le gradient à une densité de ~ 1,18 g/ml ; et
e) extraire des exosomes à partir de la fraction collectée ;
permettant ainsi d'isoler l'exosome de cellules souches mésenchymateuses (CSM).

2. - Composition pharmaceutique selon la revendication 1, dans laquelle l'exosome isolé à partir de CSM comprend en outre un ou plusieurs marqueurs choisis dans le groupe consistant en : FLOT1, CD9 et CD63.

3. - Composition pharmaceutique selon l'une des revendications 1 et 2, dans laquelle l'exosome isolé à partir des CSM :
(i) est isolé à partir d'un milieu conditionné de CSM ;
(ii) provient de la Gelée de Warton ou de la moelle osseuse ;
(iii) est sensiblement exempt de contaminants protéiques non-exosomaux ;
(iv) a un diamètre d'environ 30-150 nm ; et/ou
(v) a une activité immunomodulatrice.

4. - Composition pharmaceutique selon l'une quelconque des revendications 1 à 3, comprenant en outre :
i) un agent secondaire ; et/ou
ii) un support pharmaceutiquement acceptable.

5. - Composition pharmaceutique selon la revendication 4, dans laquelle l'agent secondaire est un surfactant pulmonaire, un stéroïde, un antioxydant ou de l'oxyde nitrique inhalé.

6. - Composition pharmaceutique selon l'une quelconque des revendications 1 à 5, pour une utilisation dans le traitement d'une lésion pulmonaire induite par hyperoxie, de l'hypertension pulmonaire induite par hyperoxie, de la dysplasie broncho-pulmonaire et dans la réduction de la fibrose pulmonaire chez un sujet.

7. - Composition pharmaceutique pour utilisation selon la revendication 6, dans laquelle le sujet est un sujet humain.

8. - Composition pharmaceutique pour utilisation selon la revendication 7, dans laquelle :
i) le sujet humain était né avant 37 semaines de grossesse ou était né avant 26 semaines de grossesse ;
ii) le sujet présente une lésion pulmonaire induite par hyperoxie ;
iii) le sujet a reçu de l'oxygène ou a été placé sous respirateur ;
iv) le sujet présente ou est à risque de développer une dysplasie bronchopulmonaire (DBP) ;
v) le sujet présente une hypertension pulmonaire induite par hyperoxie ;
vi) le sujet présente un remodelage artériel pulmonaire périphérique ;
vii) le sujet présente une inflammation dans le poumon ; et/ou
viii) le sujet est âgé de moins de quatre semaines, de quatre semaines à 3 ans, de 3 à 18 ans ; ou est un adulte.

9. - Composition pharmaceutique pour utilisation selon la revendication 7 ou 8, dans laquelle :
i) l'exosome isolé à partir de CSM est administré dans une dose de bolus au sujet ou l'exosome isolé à partir de CSM est administré de façon répétée au sujet ;
ii) l'exosome isolé à partir de CSM améliore la fonction pulmonaire ;
iii) l'exosome isolé à partir de CSM restaure l'architecture pulmonaire ;
v) l'exosome isolé à partir de CSM réduit l'inflammation dans le poumon ; et/ou
vi) l'exosome isolé à partir de CSM réduit le remodelage artériel pulmonaire périphérique.

10. - Composition pharmaceutique pour utilisation selon la revendication 9, dans laquelle l'exosome isolé à partir de CSM est administré dans l'heure qui suit la naissance, ou dans laquelle l'exosome isolé à partir de CSM est administré dans le mois qui suit la naissance.

11. - Composition pharmaceutique pour utilisation selon l'une quelconque des revendications 7 à 10, dans laquelle :
i) l'exosome isolé à partir de CSM est administré par voie intraveineuse ou intranasale ;
ii) l'exosome isolé à partir de CSM est administré au poumon ou à la trachée du sujet ;
iii) l'exosome isolé à partir de CSM est administré par inhalation ;
iv) l'exosome isolé à partir de CSM est administré dans un aérosol ;
v) l'exosome isolé à partir de CSM est administré à l'aide d'un nébuliseur ; et/ou
vi) l'exosome isolé à partir de CSM est administré à l'aide d'un tube intratrachéal.

12. - Composition pharmaceutique pour utilisation selon la revendication 6, dans laquelle le sujet est un mammifère.

13. - Composition pharmaceutique pour utilisation selon la revendication 12, dans laquelle
i) le mammifère est un rongeur, en outre facultativement le rongeur étant une souris ou un rat.

14. - Procédé d'isolement d'un exosome à partir de cellules souches mésenchymateuses (CSM), le procédé comprenant le fractionnement d'un milieu conditionné de CSM à l'aide d'un gradient coussin d'iodixanol (IDX) et la collecte d'une fraction qui est sensiblement exempte de contaminants protéiques non-exosomaux selon le procédé caractérisé dans la revendication 1.

15. - Procédé selon la revendication 14, dans lequel le milieu conditionné de CSM est concentré avant le fractionnement.

16. - Procédé selon la revendication 15, dans lequel le milieu conditionné de CSM est concentré par filtration à flux tangentiel.

17. - Procédé selon l'une quelconque des revendications 14 à 16, dans lequel le fractionnement comprend une flottaison du milieu conditionné de CSM sur le gradient coussin d'IDX et une ultracentrifugation.
